# EUROPÄISCHE PATENTANMELDUNG

(11) **EP 2 607 900 A1**
(43) Veröffentlichungstag der Anmeldung: **26.06.2013**
(21) Anmeldenummer: 11195459.0
(22) Anmeldetag: 22.12.2011
(51) Int. Cl.: G01N 33/574

(54) **Markersequenzen für Brustkrebs und deren Verwendung**

(71) Anmelder: Protagen AG, 44227 Dortmund (DE)
(72) Erfinder: Lücking, Angelika, 44892 Bochum (DE); Kowald, Axel, 44892 Bochum (DE); Höpner, Annabel, 33615 Bielefeld (DE); Scheer, Christian, 44179 Dortmund (DE); Fiegl, Heidelinde, 6082 Ellbögen (AT); Daxenbichler, Günter, 6020 Innsbruck (AT)
(74) Vertreter: Simandi, Claus

(57) **Zusammenfassung**

Die vorliegende Erfindung betrifft Brustkrebs-spezifische Markersequenzen und deren diagnostische Verwendung, eine diagnostische Vorrichtung enthaltend Markersequenzen für Brustkrebs insbesondere eine Anordnung und einen Proteinbiochip sowie deren Verwendung. Ferner betrifft die Erfindung Verfahren zur Identifizierung von Brustkrebs-spezifischen Markersequenzen und zum Screenen von potentiellen Wirkstoffen zur Behandlung und Prävention von Brustkrebs mittels dieser Markersequenzen.

## Beschreibung

Die vorliegende Erfindung betrifft Brustkrebs-spezifische Markersequenzen und deren diagnostische Verwendung, eine diagnostische Vorrichtung enthaltend Markersequenzen für Brustkrebs insbesondere eine Anordnung und einen Proteinbiochip sowie deren Verwendung. Ferner betrifft die Erfindung Verfahren zur Identifizierung von Brustkrebs-spezifischen Markersequenzen und zum Screenen von potentiellen Wirkstoffen zur Behandlung und Prävention von Brustkrebs mittels dieser Markersequenzen.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig.

Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Brustkrebs (Mammakarzinom) ist der häufigste bösartige Tumor der Brustdrüse des Menschen. Er kommt hauptsächlich bei Frauen vor; nur etwa jede hundertste dieser Krebserkrankungen tritt bei Männern auf. In den westlichen Staaten ist Brustkrebs die häufigste Krebsart bei Frauen und an Brustkrebs sterben mehr Frauen als an irgendeiner anderen Krebserkrankung. Die meisten Erkrankungen treten sporadisch (zufällig) auf, es gibt aber sowohl erbliche als auch erworbene Risikofaktoren. Zahlreiche nationale und internationale Programme zur Früherkennung und zur strukturierten Behandlung sollen die Sterblichkeit senken.

Etwa 80 bis 90 % aller Geschwulste in der weiblichen Brust werden von den Frauen selbst zufällig entdeckt. Diese tast- und sichtbaren Tumoren sind bei ihrer Entdeckung oft schon relativ groß und sind deshalb meist mit einer schlechten Prognose verbunden. Durch konsequente Früherkennung kleinerer, nicht tastbarer Tumoren könnte die Sterblichkeit deutlich gesenkt werden. Zur Früherkennung dienen Programme zur systematischen Selbstuntersuchung, der Tastuntersuchung durch den Arzt und die Screening-Mammographie mit Hilfe von Bildgebenden Verfahren sowie die Biopsie.

Bei Brustkrebs ist eine frühzeitige Diagnose für den weiteren Krankheitsverlauf und die Prognose entscheidend. Obwohl zahlreiche Tumormarker mit Brustkrebs assoziiert sind, sind diese nicht zur Früherkennung geeignet, da sie eine zu geringe Spezifität aufweisen. Sie werden vorwiegend zur Überwachung des Krankheitsverlaufs bzw. des Ansprechens auf die Therapie verwendet.

Es besteht deshalb ein Bedürfnis an indikationsspezifischen diagnostischen Vorrichtungen und Verfahren für Brustkrebs. Es ist die Aufgabe der vorliegenden Erfindung verbesserte Mittel zur Früherkennung und Therapiesteuerung bei Brustkrebs bereit zu stellen.

Die Erfindung betrifft eine Anordnung von Brustkrebs-spezifischen Markersequenzen auf einem Träger zur Früherkennung, Diagnose, Prognose, Therapiesteuerung bei Brustkrebs mit einem oder mehreren verschiedenen Brustkrebs-spezifische Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 (Klon Sequenzen) und/oder kodiert durch Sequenzen SEQ ID. No. 492 - 982 (RNA Sequenzen) und/oder kodiert durch Teilsequenzen von SEQ ID. No. 1 - 982.

Gegenstand der Erfindung ist auch eine Anordnung von Brustkrebs-spezifischen Markersequenzen auf einem Träger zur Analyse von Brustkrebs-assoziierten Autoantikörperprofilen mit einem oder mehreren verschiedenen Brustkrebs-spezifischen Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID. No. 1 - 982.

Im Rahmen diese Erfindung wurde für die Proteine SEQ ID No. 983 bis 1473 und/oder Teilsequenzen dieser Proteine und/oder Proteine kodiert durch Sequenzen SEQ ID No. 1 - 491 und/oder Proteine kodiert durch Sequenzen SEQ ID. No. 492 - 982 und/oder kodiert durch Teilsequenzen von SEQ ID. No. 1 - 982 erstmals eine Brustkrebs-spezifische Verwendung gefunden und in der erfindungsgemäßen Anordnung umgesetzt. Die Erfindung stellt damit ein Panel von Brustkrebs-spezifischen Markersequenzen zur Verfügung, die Rahmen einer individualisierten Diagnostik und Therapie verwendet werden können, um bei unterschiedlichen Patienten, Patientengruppen, Kohorten, Bevölkerungsgruppen, Brustkrebsvarianten u.s.w. gezielt und individuell angepasst Brustkrebs zu diagnostizieren und die Therapie zu überwachen.

Gegenstand der Erfindung ist auch die Verwendung von einer oder mehreren Brustkrebs-spezifischen Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID. No. 1 - 982 für Brustkrebsdiagnose und Therapie, insbesondere zur Früherkennung von Brustkrebs, zur Diagnose von Brustkrebs, zur Prognose, beispielsweise des Risikos der Metastasenbildung, Therapiesteuerung, beispielsweise Vorhersage und Überwachung des Ansprechens auf ein Arzneimittel oder eine Therapie, Nachsorge. Die Erfindung betrifft insbesondere auch den Nachweis und die Bestimmung der Menge von mindestens zwei verschiedenen Autoantikörpern bei einem Patienten wobei entsprechend mindestens zwei verschiedene erfindungsgemäße Brustkrebs-spezifische Markersequenzen (als Antigene) verwendet werden.

In bevorzugten Ausführungsformen der Erfindung umfasst die erfindungsgemäße Anordnung/Verwendung 2 oder 3, vorzugsweise 4 oder 5, besonders bevorzugt 7 oder 8 oder mehr unterschiedliche Brustkrebs-spezifische Markersequenzen. Die Anordnung/Verwendung kann 9 oder 10 oder mehr Brustkrebs-spezifische Markersequenzen umfassen, beispielsweise 10 bis 50, vorzugsweise 50 bis 100 oder mehr. Erfindungsgemäß umfasst sind auch Anordnungen/Verwendungen, die für Patienten individuell hergestellt werden, beispielsweise im Rahmen der individualisierten (personalisierten) Medizin. Gegenstand der Erfindung ist somit auch eine Anordnung/Verwendung wobei mindestens 2 bis 5 oder 10, vorzugsweise 30 bis 50 Brustkrebs-spezifische Markersequenzen oder 50 bis 100 oder mehr Brustkrebs-spezifische Markersequenzen an oder zu einem zu untersuchenden Patienten bestimmt werden.

Eine bevorzugte Ausführungsform der Erfindung betrifft eine Anordnung/Verwendung, dadurch gekennzeichnet, dass die Brustkrebs-spezifischen Markersequenzen auf einem festen Träger aufgebracht werden, insbesondere einem Filter, einer Membran, einem magnetisches oder Fluorophor-markiertes Kügelchen, einem Silizium-Wafer, Glas, Metall, Kunststoff, einem Chip, einem massenspektrometrisches Target oder einer Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

Eine weitere Ausführungsform betrifft eine Anordnung/Verwendung, dadurch gekennzeichnet, dass die Brustkrebs-spezifischen Markersequenzen als Klone vorliegen. Daher betrifft die Erfindung die Verwendung von Brustkrebs-spezifischen Markersequenzen zur Diagnose von Brustkrebs, wobei mindestens eine Brustkrebs-spezifische Markersequenz einer DNA, insbesondere cDNA ausgewählt aus der Gruppe SEQ ID No. 1-491 oder RNA ausgewählt aus der Gruppe 492-982 oder einer Teilsequenz oder eines Fragments davon an oder zu einem zu untersuchenden Patienten bestimmt wird.

Die Bereitstellung von Brustkrebs-spezifischen Markersequenzen (nachstehend auch erfindungsgemäße Markersequenzen genannt) erlaubt eine sichere Diagnose und Stratifizierung von Patienten mit Brustkrebs insbesondere mittels eines Proteinbiochips.

Die erfindungsgemäßen Brustkrebs-spezifischen Markersequenzen konnten mittels differentiellem Screenen von Proben und zwar gesunder Probanden mit Patientenproben mit Brustkrebs identifiziert werden. Hierbei konnten diese erfindungsgemäßen Markersequenzen erstmals mittels Proteinbiochips (siehe Beispiele) identifiziert werden.

Gegenstand der Erfindung ist auch ein Verfahren zur Identifizierung von Brustkrebs-spezifischen Markersequenzen umfassend die Schritte
a) Bereitstellung von Markersequenzen auf einem Array,
b) Identifizierung von Brustkrebs-spezifischen Markersequenzkandidaten durch vergleichende Analyse der Signale, die bei Kontakt der Markersequenzen mit Körperflüssigkeit oder Gewebeauszug eines Patienten mit Brustkrebs und Körperflüssigkeit oder Gewebeauszug eines Patienten ohne Brustkrebs gemessen werden,
c) Charakterisierung der Brustkrebs-spezifischen Markersequenzkandidaten mit Hilfe eines Proteinbiochips,
d) Auswahl von Brustkrebs-spezifischen Markersequenzenkandidaten, die ein unterschiedliches Signal bei Patienten mit Brustkrebs und Patienten ohne Brustkrebs liefern (Brustkrebs-spezifische Markersequenzen).

Der Begriff "Brustkrebs" umfasst eine Gruppe von Erkrankungen, die Vorstufen zu Brustkrebs sein können und deren Etablierung als Brustkrebs, Mammakarzinom (Definition z.B. nach Pschyrembel, de Gruyter, 263. Auflage (2012), Berlin).

In einer weiteren Ausführungsform der Erfindung können die erfindungsgemäßen Markersequenzen ebenfalls mit bekannten Biomarkern für diese Indikation kombiniert, ergänzt oder erweitert werden. Dabei sind jedoch mindestens 50 %, vorzugsweise 60 %, besonders bevorzugt 70 % oder mehr erfindungsgemäße Markersequenzen vertreten, beispielsweise in der erfindungsgemäßen Anordnung. In besonders bevorzugten Ausführungsformen der Erfindung, insbesondere der erfindungsgemäßen Anordnung, dem erfindungsgemäßen Assay und Proteinbiochip sowie der erfindungsgemäßen Verwendung sind mindestens 75 %, vorzugsweise 80 % oder 85 %, besonders bevorzugt 90 % oder 95 % erfindungsgemäße Markersequenzen vertreten.

In einer bevorzugten Ausführungsform erfolgt die Bestimmung der Brustkrebs-spezifischen Markersequenzen außerhalb des menschlichen Körpers und die Bestimmung erfolgt in einer ex vivo / in vitro Diagnose.

Gegenstand der Erfindung ist auch ein Assay oder Proteinbiochip umfassend eine erfindungsgemäße Anordnung/Verwendung. Die Erfindung betrifft eine diagnostische Vorrichtung und/oder einen Assay, insbesondere einen Proteinbiochip der für Brustkrebs eine Früherkennung, Diagnose, Prognose, Stratifizierung und/oder Untersuchung erlaubt.

Die Erfindung betrifft auch die Verwendung einer erfindungsgemäßen Anordnung oder eines erfindungsgemäßen Assays oder Proteinbiochips zur Analyse von Autoantikörperprofilen von Patienten, insbesondere zur quantitativen Analyse und/oder zur Überwachung von Veränderungen von Autoantikörperprofilen von Patienten.

Gegenstand der Erfindung ist auch ein Diagnostikum (Test Kit) zur Früherkennung und/oder Diagnose von Brustkrebs und/oder Prognose und/oder Vorhersage des Risikos der Metastasenbildung bei Brustkrebs umfassend eine erfindungsgemäße Anordnung vorzugsweise auf einem Träger oder einen erfindungsgemäßen Assay oder Proteinbiochip und gegebenenfalls weitere Zusatz- und Hilfsstoffe.

Gegenstand der Erfindung ist auch ein Diagnostikum (Test Kit) zur Therapieüberwachung und/oder Nachsorge bei Brustkrebs umfassend eine erfindungsgemäße Anordnung oder einen erfindungsgemäßen Assay oder Proteinbiochip und gegebenenfalls weitere Zusatz- und Hilfsstoffe.

In einer weiteren Ausführungsform der Erfindung betrifft die Erfindung die Verwendung von Brustkrebs-spezifischen Markersequenzen als Diagnostika, wobei mindestens eine Markersequenz einer cDNA ausgewählt aus der Gruppe SEQ ID No. 1-491 (Klon Sequenzen) oder SEQ ID No. 492-982 (RNA) oder jeweils ein durch SEQ ID No. 1-982 kodiertes Protein oder jeweils eine Teilsequenz oder Fragment davon ist.

Gegenstand der Erfindung ist auch ein Verfahren zur Früherkennung und Diagnose von Brustkrebs wobei
a.) eine oder mehrere Brustkrebs-spezifische Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID No. 1 - 982 auf einem Träger aufgebracht werden und
b.) mit Körperflüssigkeit oder Gewebeauszug eines Patienten in Kontakt gebracht werden und
c.) der Nachweis einer Wechselwirkung der Körperflüssigkeit oder Gewebeauszug mit den Brustkrebs-spezifischen Markersequenzen aus a.) erfolgt.

Ferner betrifft die Erfindung ein Verfahren zur Früherkennung und Diagnose von Brustkrebs wobei
a.) mindestens eine Brustkrebs-spezifische Markersequenz einer cDNA ausgewählt aus der Gruppe SEQ ID No. 1-491 (Klon Sequenzen) oder SEQ ID No. 492-982 (RNA) oder eine Teilsequenz von SEQ ID. No. 1-982 auf einen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

Eine besondere Ausführungsform der Erfindung betrifft Verfahren zur Früherkennung und Diagnose von Brustkrebs, wobei die Wechselwirkung nach c.) ein Brustkrebs-assoziertes Autoantikörperprofil des Patienten oder einer Kohorte oder einer Bevölkerungsgruppe oder eines bestimmten Krankheitsverlaufs (Prognose) oder eines bestimmten Ansprechens auf eine Therapie/Arzneimittel abbildet.

In einem Verfahren zur Diagnose und/oder in einem Diagnostikum werden ein oder mehrere Brust-krebs-spezifische Markersequenzen verwendet. In einer bevorzugten Ausführungsform werden mindestens 2, beispielsweise 3, 4, 5, 6, 7, 8, 9, 10, vorzugsweise 15 bis 20 Markersequenzen oder 30 bis 50 oder 100 oder mehr Brustkrebs-spezifische Markersequenzen zusammen oder in Kombination verwendet, beispielsweise direkt nacheinander oder parallel.

Der Nachweis einer Wechselwirkung der Körperflüssigkeit oder des Gewebeauszugs mit der oder den Brustkrebs-spezifischen Markersequenzen kann beispielsweise durch eine Sonde, insbesondere durch einen Antikörper erfolgen.

Gegenstand der Erfindung ist auch ein Verfahren zum Stratifizieren, insbesondere zur Risikostratifizierung, oder zur Therapiesteuerung eines Patienten mit Brustkrebs, wobei mit Hilfe einer oder mehrerer Brustkrebs-spezifischer Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID No. 1 - 982 das Brustkrebsassoziierte Autoantikörperprofil eines Patienten bestimmt und gegebenenfalls überwacht wird.

Eine besondere Ausführungsform der Erfindung betrifft das Verfahren, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

Ferner betrifft die Erfindung ein Verfahren zum Stratifizieren, insbesondere zur Risikostratifizierung oder Therapiesteuerung eines Patienten mit Brustkrebs, wobei mindestens eine Markersequenz einer Nuleinsäure, beispielsweise cDNA ausgewählt aus der Gruppe SEQ ID No. 1-491 (Klon Sequenzen) oder SEQ ID No. 492-982 (RNA) oder jeweils ein dadurch kodiertes Protein oder jeweils eine Teilsequenz von SEQ ID No. 1 - 982 an einem zu untersuchenden Patienten bestimmt wird.

Ferner betrifft die Erfindung ein Verfahren zum Stratifizieren, insbesondere zur Risikostratifizierung und / oder Therapiesteuerung eines Patienten mit Brustkrebs, wobei mindestens eine Markersequenz einer DNA, cDNA ausgewählt aus der Gruppe SEQ ID No. 1-491 (Klon Sequenzen) oder SEQ ID No. 492-982 (RNA bzw. DNA) oder jeweils eine Teilsequenz davon verwendet wird um Brustkrebs-spezifische Autoantikörper und/oder Brustkrebs-spezifische Autoantikörperprofile bei dem Patienten nachzuweisen, zu identifizieren, zu überwachen.

Autoantikörperprofile umfassen die Menge an einem oder mehreren Autoantikörpern deren Vorkommen/Expression mit der Entstehung und/oder Etablierung von Brustkrebs einhergehen. Ferner umfasst ist die Stratifizierung der Patienten mit Brustkrebs in neue oder etablierte Subgruppen der Brustkrebs Patienten, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung von Brustkrebs mittels der erfindungsgemäßen Brustkrebs-spezifischen Markersequenzen sowie die Zuordnung der Patienten zu Brustkrebs. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von Brustkrebs mittels der erfindungsgemäßen Brustkrebs-spezifischen Markersequenzen sowie die Prognose von Brustkrebs, insbesondere die Vorhersage des Risikos der Metastasenbildung.

"Stratifizieren (auch: Stratifikation) oder Therapiesteuerung" im Sinne dieser Erfindung bedeutet, dass beispielsweise die erfindungsgemäßen Verfahren Entscheidungen zur Behandlung und Therapie des Patienten erlauben, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

"Prognose" bedeutet die Vorhersage des Krankheitsverlaufs, beispielsweise die Vorhersage des Rezidiv-freien Überlebens, des Gesamtüberlebens, des Risikos der Metastasenbildung.

Im Rahmen dieser Erfindung wird unter "Patient" ein beliebiger Proband - Mensch oder Säugetier - verstanden, mit der Maßgabe, dass der Proband auf Brustkrebs untersucht wird.

Der Begriff "Brustkrebs-spezifische Markersequenz(en)" im Sinne dieser Erfindung bedeutet, dass die Nukleinsäure, beispielsweise DNA, insbesondere cDNA oder RNA oder die kodierte Aminosäuresequenz oder das jeweils daraus erhältliche Polypeptid oder Protein signifikant (spezifisch) für Brustkrebs sind. Brustkrebs-spezifische Markersequenzen können Nukleinsäuresequenzen und Aminosäuresequenzen sein, wobei auch Modifikationen umfasst sind.

"Brustkrebs-spezifisch" bedeutet, dass beispielsweise die cDNA oder das jeweils daraus erhältliche Polypeptid oder Protein eine Wechselwirkung mit Substanzen aus der Körperflüssigkeit oder Gewebeauszug eines Patienten mit Brustkrebs aufweisen (z.B. Antigen (Epitop) / Antikörper (Paratop) Wechselwirkung). Diese Substanzen aus der Körperflüssigkeit oder Gewebeauszug treten entweder nur oder zumindest verstärkt bei Brustkrebs auf beziehungsweise werden exprimiert, wohingegen diese Substanzen bei Patienten ohne Brustkrebs nicht oder zumindest in geringerem Maße (geringere Menge, geringere Konzentration) vorhanden sind. Brustkrebs-spezifische Markersequenzen können sich andererseits auch dadurch auszeichnen, dass sie eine Wechselwirkung mit Substanzen aus der Körperflüssigkeit oder Gewebeauszug von Patienten mit Brustkrebs eingehen, weil diese Substanzen nicht mehr oder zumindest in deutlich geringerer Menge/Konzentration bei Brustkrebs auftreten beziehungsweise exprimiert werden, wohingegen diese Substanzen bei Patienten ohne Brustkrebs vorhanden oder zumindest in deutlich höherem Maße vorhanden sind. Brustkrebs-spezifische Markersequenzen können auch in gesunden Probanden vorhanden sein, jedoch verändert sich ihre Menge (Konzentration) beispielsweise bei der Entstehung, Etablierung und Therapie von Brustkrebs. Die Brustkrebs-spezifischen Markersequenzen sind deshalb Biomarker für Brustkrebs. Die Brustkrebs-spezifischen Markersequenzen können auf diese Weise ein Profil von Substanzen aus Körperflüssigkeit und Gewebeauszug abbilden, beispielsweise ein Brustkrebs-assoziiertes Autoantikörperprofil.

"Brustkrebs-assoziierte Autoantikörperprofile" umfassen somit einerseits die Zusammensetzung (ein oder mehrere Autoantikörper) und anderseits die Menge/Konzentration einzelner Autoantikörper.

In einer besonders bevorzugten Ausführungsform der Erfindung ist die Brustkrebs-spezifische Markersequenz ein Antigen oder ein Teil eines Antigens oder kodiert für ein Antigen oder für einen Teil eines Antigens.

In einer besonders bevorzugten Ausführungsform erkennt/bindet die Brustkrebs-spezifische Markersequenz an Autoantikörper, die im Verlauf der Entstehung, Etablierung und Therapie von Brustkrebs (verstärkt) vorhanden sind oder in geringerem Maße (oder nicht mehr) vorhanden sind (nachfolgend "Brustkrebsassoziierte Autoantikörper"). Autoantikörper werden vom Körper gegen körpereigene Antigene, die beispielsweise bei Brustkrebs entstehen, gebildet. Autoantikörper werden von Körper gegen unterschiedliche Substanzen und Pathogenen gebildet. Im Rahmen der vorliegenden Erfindung werden insbesondere die Brustkrebs-assoziierten Autoantikörper detektiert, die beim Auftreten und im Laufe der Entstehung von Brustkrebs gebildet werden und/oder in ihrer Expression hoch- beziehungsweise herunterreguliert werden. Brustkrebs-assozierte Autoantikörper können mit Hilfe der erfindungsgemäßen Verfahren und Brustkrebs-spezifischen Markersequenzen nachgewiesen werden und somit als Indikation für Brustkrebs dienen. Der Nachweis und die Überwachung der Menge an Brustkrebs-assozierten Autoantikörpern im Patienten kann zur Früherkennung, Diagnose und/oder Therapieüberwachung/Therapiesteuerung verwendet werden. Diese Brustkrebs-assoziierten Autoantikörperprofile können bereits bei Verwendung von einer Brustkrebs-spezifischen Markersequenz ausreichend charakterisiert werden. In anderen Fällen werden zwei oder mehr Brustkrebs-spezifische Markersequenzen notwendig sein, um ein Brustkrebs-assoziiertes Autoantikörperprofil abzubilden.

In bevorzugten Ausführungsformen der Erfindung können die Brustkrebs-assozierten Autoantikörper mit Brustkrebs-spezifischen Markersequenzen nachgewiesen werden, die sich von einem anderen Individuum ableiten, weil sie beispielsweise aus einer kommerziellen cDNA-Bank stammen. Bevorzugte Ausführungsformen der Erfindung betreffen die Brustkrebs-assozierten Markersequenzen SEQ ID No. 1 - 491, SEQ ID. No. 492 - 982 und/oder Teilsequenzen von SEQ ID No. 1 - 982, und Sequenzen, die für die Proteine SEQ ID No. 983 bis 1473 und/oder Teilsequenzen dieser Proteine kodieren.

In anderen bevorzugten Ausführungsformen der Erfindung können die Brustkrebs-assozierten Autoantikörper mit Brustkrebs-spezifischen Markersequenzen nachgewiesen werden, die sich von dem gleichen Individuum ableiten (Autoantigen), weil sie beispielsweise aus einer eigens für den Patienten oder eine Gruppe von Patienten (beispielsweise im Rahmen der individualisierten Medizin) hergestellten cDNA-Bank stammen. Beispielsweise werden dann Homologe der genannten Brustkrebs-spezifischen Markersequenzen SEQ ID. No. 1 - 1473 oder Teilsequenzen davon verwendet.

Autoantikörper können bereits viele Jahre vor Auftreten der ersten Krankheitssymptome vom Patienten gebildet werden. Damit wäre eine Früherkennung, Diagnose und auch Prognose und (vorbeugende) Behandlung bereits Jahre vor dem sichtbaren Krankheitsausbruch möglich. Die erfindungsgemäßen Vorrichtungen und Mittel (Anordnung, Array, Proteinbiochip, Diagnostikum, Test Kit) und Verfahren ermöglichen somit ein im Vergleich zu bekannten Verfahren sehr frühes Eingreifen, was die Prognose und Überlebensraten deutlich verbessert. Da sich die Brustkrebs-assoziierten Autoantikörperprofile während der Etablierung und Behandlung/Therapie von Brustkrebs verändern, ermöglicht die Erfindung auch den Nachweis und die Überwachung von Brustkrebs in jedem Stadium der Entstehung und Behandlung sowie die Überwachung im Rahmen der Brustkrebsnachsorge. Die erfindungsgemäßen Mittel erlauben auch eine einfache Handhabung zu Hause durch den Patienten und die kostengünstige routinemäßige Vorsorge zur Früherkennung.

Insbesondere durch die Verwendung von Antigenen als spezifische Markersequenz für Brustkrebs, die sich von bereits bekannten Sequenzen, z.B. aus kommerziellen cDNA Banken, ableiten, können Probanden getestet und gegebenenfalls vorhandene Brustkrebs-assoziierte Autoantikörper in diesen Probanden nachgewiesen werden, auch wenn bei diesen Probanden die entsprechenden Autoantigene (noch) nicht bekannt sind.

Verschiedene Patienten können unterschiedliche Brustkrebs-assozierte Autoantikörperprofile aufweisen, beispielsweise unterscheiden sich verschiedene Kohorten oder Bevölkerungsgruppen. Jeder Patient kann dabei ein oder mehrere verschiedene Brustkrebs-assozierte Autoantikörper im Laufe der Entstehung von Brustkrebs und des Fortschreitens der Brustkrebserkrankung, d.h. ebenfalls verschiedene Autoantikörperprofile, bilden. Außerdem kann sich die Zusammensetzung und/oder die Menge der gebildeten Brustkrebs-spezifischen Autoantikörper im Laufe der Brustkrebs Entstehung und des Fortschreitens der Krankheit verändern, so dass eine quantitative Auswertung notwendig wird. Auch die Therapie/Behandlung von Brustkrebs führt zu Veränderungen in der Zusammensetzung und/oder der Menge an Brustkrebs-assoziierten Autoantikörpern. Die große Auswahl an erfindungsgemäßen Brustkrebs-spezifischen Markersequenzen ermöglichen die individuelle Zusammenstellung von Brustkrebs-spezifischen Markersequenzen in einer Anordnung für einzelne Patienten, Gruppen von Patienten, bestimmte Kohorten, Bevölkerungsgruppen und so weiter. Im Einzelfall kann deshalb die Verwendung einer Brustkrebs-spezifischen Markersequenz ausreichen, während in anderen Fällen mindestens zwei oder mehr Brustkrebs-spezifische Markersequenzen zusammen oder in Kombination verwendet werden müssen um ein aussagekräftiges Autoantikörperprofil zu erstellen.

Der Nachweis von Brustkrebs-assoziierten Autoantikörpern beispielsweise im Serum/Plasma hat gegenüber anderen Biomarkern den Vorteil einer hohen Stabilität und Lagerfähigkeit und einer guten Nachweisbarkeit. Auch unterliegt die Anwesenheit von Autoantikörpern keinem circardianen Rhythmus, so dass die Probennahme unabhängig von Tageszeit, Nahrungsaufnahme und dergleichen ist.

Daneben können die Brustkrebs-assozierten Autoantikörper mit Hilfe der korrespondierenden Antigene/Autoantigene in bekannten Assays wie z.B. ELISA oder Western-Blot nachgewiesen werden und auf dieses die Ergebnisse überprüft werden.

Im Sinne der Erfindung bedeutet "wobei mindestens eine Brustkrebs-spezifsche Markersequenz ausgewählt wird", dass eine Wechselwirkung nachgewiesen wird. Eine solche Wechselwirkung ist z.B. eine Bindung, insbesondere eine bindende Substanz an mindestens eine Brustkrebs-spezifische Markersequenz oder im Fall, dass die Brustkrebs-spezifische Markersequenz eine Nukleinsäure, beispielsweise eine cDNA ist, die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Die Wechselwirkung zwischen der Körperflüssigkeit oder Gewebeauszuges eines Patienten und den Brustkrebs-spezifischen Markersequenzen ist vorzugsweise eine Protein-Protein Wechselwirkung.

Solche Substanzen, beispielsweise Antigene, Autoantigene, Brustkrebs-assozierte Autoantikörper, sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges, beispielsweise aus Tumorgewebe des Patienten. Die Erfindung betrifft insbesondere die Verwendung dieser Körperflüssigkeiten und Gewebeauszüge für Früherkennung, Diagnose, Prognose, Therapiesteuerung und Nachsorge.

In einer weiteren Ausführungsform der Erfindung können jedoch die Brustkrebs-spezifischen Markersequenzen bzw. die von diesen Markersequenzen erkannten Substanzen, beispielsweise Brustkrebs-assoziierte Autoantikörper, in einer signifikant höheren oder niedrigeren Expressionsrate oder Konzentration vorliegen, was auf Brustkrebs hinweist. Hierbei werden mittels Proteomics oder Nukleinsäureblots die relativen Expressionsraten krank / gesund der erfindungsgemäßen Markersequenzen für Brustkrebs bzw. die von diesen Markersequenzen erkannten Substanzen bestimmt.

Die Brustkrebs-spezifischen Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Brustkrebs-spezifischen Markersequenzen sind Gegenstand der Tabelle A (RNA) und Tabelle B (Proteinsequenzen) und können durch den jeweilig zitierten Datenbankeintrag (auch mittels Internet:
http://www.ncbi.nlm.nih.gov/) eindeutig identifiziert werden (mittels Accession No.), siehe ebenfalls das zugehörige Sequenzprotokoll. Die Klonsequenzen (cDNA) sind nur im anliegenden Sequenzprotokoll zu finden.

Daher umfasst die Erfindung ebenfalls die Volllängesequenzen der erfindungsgemäßen Brustkrebs-spezifischen Markersequenzen und zwar wie über den bekannten Datenbankeintrag gemäß Tabellen A, B, C definiert, nachstehend SEQ 1 - 1473 genannt.

Weiterhin umfasst sind daher ebenfalls analoge Ausführungsformen von SEQ 1 -1473 zu den Brustkrebs-spezifischen Markersequenzen SEQ 1-1473, wie z.B. in den Ansprüchen dargelegt, da die erfindungsgemäßen SEQ 1-1473 wiederum Teilsequenzen, zumindest mit hoher Homologie, darstellen. Die Brustkrebs-spezifischen Markersequenzen SEQ 1-1473 sind jedoch erfindungsgemäß bevorzugt.

Erfindungsgemäß umfassen die Markersequenzen auch solche Modifikationen der Nukleinsäuresequenz, insbesondere cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

Die Erfindung betrifft auch Homologe der Brustkrebs-spezifischen Markersequenzen und Teilsequenzen, beispielsweise Fragmente von Brustkrebs-spezifischen Markersequenzen. Homologe sind beispielsweise Nukleinsäure- und/oder Proteinsequenzen, insbesondere Homologe von SEQ ID No. 1 - 1473, die eine Identität mit den Brustkrebs-spezifischen Markersequenzen von mindestens 70 % oder 80 %, vorzugsweise 90 % oder 95 %, besonders bevorzugt 96 % oder 97 % oder mehr, beispielsweise 98 % oder 99 % aufweisen. In einer besonders bevorzugten Ausführungsform der Erfindung beträgt für den Fall, dass es sich bei den Brustkrebs-spezifischen Markersequenzen um Antigene handelt, die Homologie im Sequenzbereich in dem die Antigen-Antikörper- beziehungsweise Antigen-Autoantikörper-Wechselwirkung stattfindet mindestens 95 %, vorzugsweise mindestens 97 %, besonders bevorzugt mindestens 99 %.

Gegenstand der Erfindung sind auch Teilsequenzen der Brustkrebs-spezifischen Markersequenzen. Teilsequenzen umfassen auch Fragmente der erfindungsgemäßen Markersequenzen, Teilsequenzen sind solche Nukleinsäuren oder Proteine/Peptide, die gegenüber der vollständigen Nukleinsäure oder dem vollständigen Protein/Peptid verkürzt sind. Die Deletion kann sich dabei an dem oder den Ende und/oder innerhalb der Sequenz befinden. Umfasst sind beispielsweise Teilsequenzen und/oder Fragmente die 50 bis 100 Nukleotide, 70-120 Nukleotide einer vollständigen Sequenz aufweisen, beispielsweise von SEQ 1-1473. Homologe von Teilsequenzen und Fragmenten sind ebenfalls erfindungsgemäß umfasst. In einer besonderen Ausführungsform sind die Brustkrebs-spezifischen Markersequenzen gegenüber den Sequenzen 1 - 1473 so weit verkürzt, dass sie nur noch aus der/den Bindungsstellen für den betreffenden Brustkrebs-assoziierten Autoantikörper bestehen. Erfindungsgemäß umfasst sind auch Brustkrebs-spezifische Markersequenzen, die sich von den Sequenzen SEQ ID No. 1 - 1473 dadurch unterscheiden, dass sie ein oder mehrere Insertionen beinhalten, wobei die Insertionen beispielsweise 1 bis 100 oder mehr Nukleotide/Aminosäuren, vorzugsweise 5 bis 50, besonders bevorzugt 10 bis 20 Nukleotide/Aminosäuren lang sind und die Sequenzen aber ansonsten identisch oder homolog zu den Sequenzen 1 - 1473 sind.

In einer weiteren Ausführungsform kann die jeweilige Brustkrebs-spezifische Markersequenz in unterschiedlichen Mengen in einem oder mehreren Bereichen auf dem Träger repräsentiert sein. Dies erlaubt eine Variation der Sensitivität. Die Bereiche können jeweils eine Gesamtheit von Brustkrebs-spezifischen Markersequenzen aufweisen, d.h. eine genügende Zahl an verschiedenen Brustkrebs-spezifischen Markersequenzen, insbesondere 2, 3, 4, 5, 6, 7, 8, 9 oder 10 oder mehr verschiedene und gegebenenfalls weitere Nukleinsäuren und/oder Proteinen, insbesondere Biomarker.

In einer besonders bevorzugten Ausführungsform der Erfindung sind mindestens 96 bis 25.000 (numerisch) oder mehr verschiedene oder gleiche Brustkrebs-spezifische Markersequenzen und gegebenenfalls weitere Nukleinsäuren und/oder Proteinen, insbesondere Biomarker auf dem Träger repräsentiert. Weiterhin bevorzugt sind mehr als 2.500, besonders bevorzugt 10.000 oder mehr verschiedene oder gleiche Brustkrebs-spezifische Markersequenzen und gegebenenfalls weitere Nukleinsäuren und/oder Proteine, insbesondere Biomarker auf dem Träger repräsentiert.

Im Rahmen dieser Erfindung bedeutet "Anordnung" synonym "Array" und sofern dieser "Array" zur Identifizierung von Substanzen an Brustkrebs-spezifischen Markersequenzen verwendet wird, ist hierunter vorzugsweise ein "Assay" oder eine diagnostische Vorrichtung zu verstehen. In einer bevorzugten Ausführungsform ist die Anordnung derart gestaltet, dass die auf der Anordnung repräsentierten Brust-krebs-spezifischen Markersequenzen in Form eines Gitters auf einem Träger vorliegen. Ferner sind solche Anordnungen bevorzugt, die eine hochdichte (high-density) Anordnung von Brustkrebs-spezifischen Markersequenzen, beispielsweise Proteinbindern erlauben. Vorzugsweise werden die Brustkrebs-spezifischen Markersequenzen gespottet. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren.

Ein "Proteinbiochip" im Sinne dieser Erfindung ist die systematische Anordnung von Brustkrebs-spezifischen Markersequenzen auf einem festen Träger, wobei die Brustkrebs-spezifischen Markersequenzen Proteine oder Peptide oder Teile davon sind.

Die Brustkrebs-spezifischen Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Brustkrebs-spezifische Markersequenzen können mehrfach in der Gesamtheit aller Brustkrebs-spezifischen Markersequenzen präsent sein und in unterschiedlichen Mengen bezogen auf einen Spot vorliegen. Ferner können die Brustkrebs-spezifischen Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

In einer weiteren Ausführungsform liegen die Brustkrebs-spezifischen Markersequenzen als Klone vor. Solche Klone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Klone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe, beispielsweise aus Brustgewebe oder Gewebe aus Mammakarzinom). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden.

Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Klone ebenfalls nicht abschließend solche sein wie transformierte Bakterien, rekombinante Phagen oder transformierte Zellen von Säugern, Insekten, Pilzen, Hefen oder Pflanzen.

Die Klone werden auf einen festen Träger fixiert, gespottet oder immobilisiert. Daher betrifft die Erfindung eine Anordnung/Verwendung, wobei die Brustkrebs-spezifischen Markersequenzen als Klone vorliegen.

Zusätzlich können die Brustkrebs-spezifischen Markersequenzen in der jeweiligen Form in Form eines Fusionsproteins vorliegen, welches beispielsweise mindestens ein Affinitätsepitop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, Calmodulin-bindendes Protein, Glutathione S-transferase oder lacZ enthalten.

In einer weiteren bevorzugten Ausführungsform der erfindungsgemäßen Anordnung/Verwendung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz (beispielsweise auch Hit, Leitsubstanz, Kandidat, Wirkstoff) für Brustkrebs, dadurch gekennzeichnet, dass eine erfindungsgemäße Anordnung oder Assay mit
a.) mindestens einer zu untersuchenden Substanz in Kontakt gebracht wird und
b.) ein Bindungserfolg nachgewiesen wird.

Die zu untersuchende Substanz kann ein beliebiges natives oder nicht-natives Biomolekül, ein (synthetisches) chemisches Molekül, ein Naturstoff, eine Mischung oder eine Substanzbibliothek sein.

Nachdem die zu untersuchende Substanz eine Brustkrebs-spezifische Markersequenz kontaktiert hat, erfolgt die Auswertung des Bindungserfolges, beispielsweise unter Verwendung mit handelsüblicher Image-Analyse Software (GenePix Pro (Axon Laboratories), Aida (Raytest), ScanArray (Packard Bioscience).

Die Visualisierung erfindungsgemäßer Bindungen, Bindungserfolge, Wechselwirkungen wie Protein-Protein-Wechselwirkungen (z.B. Protein an Brustkrebs-spezifische Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Brustkrebs entwickelt und erhältlich durch den Einsatz einer erfindungsgemäßen Brustkrebs-spezifischen Markersequenz, einer erfindungsgemäßen Anordnung, einer erfindungsgemäßen Verwendung, eines erfindungsgemäßen Assays.

Daher betrifft die Erfindung in einer weiteren Ausführungsform ein Target zur Behandlung und Therapie von Brustkrebs ausgewählt aus der Gruppe der Proteine SEQ ID No. 983 bis 1473, Teilsequenzen dieser Proteine und den für diese Proteine kodierenden Sequenzen, den Sequenzen SEQ ID No. 1 - 491 und SEQ ID. No. 492 - 982 und Teilsequenzen von SEQ ID No 1 - 982 sowie der von SEQ ID No. 1 - 982 kodierten Proteine.

Gegenstand der Erfindung ist auch die Verwendung einer Brustkrebs-spezifischen Markersequenz ausgewählt aus der Gruppe der Proteine SEQ ID No. 983 bis 1473, Teilsequenzen dieser Proteine und den für diese Proteine kodierenden Sequenzen, den Sequenzen SEQ ID No. 1 - 491 und SEQ ID. No. 492 - 982 und Teilsequenzen von SEQ ID No 1 - 982 sowie der von SEQ ID No. 1 - 982 kodierten Proteine als Affinitätsmaterial zur Durchführung einer Apherese oder Blutwäsche für Patienten mit Brustkrebs. Die Erfindung betrifft somit die Verwendung der erfindungsgemäßen Markersequenzen, vorzugsweise in Form einer Anordnung, als Affinitätsmaterial zur Durchführung einer Blutwäsche im weiteren Sinne, wobei Substanzen aus Körperflüssigkeiten eines Patienten mit Brustkrebs, wie Blut oder Plasma, an die erfindungsgemäßen Markersequenzen binden und folglich der Körperflüssigkeit selektiv entzogen werden können.

Die nachfolgenden Beispiele dienen der Erläuterung der Erfindung ohne die Erfindung jedoch auf die Beispiele einzuschränken.

Die Durchführung der Beispiele erfolgt unter Verwendung der UNIarray Technologieplattform auf Basis quantitativer Analysen der Autoantikörperprofile im Serum von Brustkrebspatientinnen. Dadurch sollen systematisch Brustkrebs-assoziierte Antigene und Brustkrebsassoziierte Autoantigene (sogenannte Biomarker) identifiziert werden, die eine Früherkennung von Brustkrebs ermöglichen und/oder auf eine bestimmte Verlaufsform hindeuten (prognostische Relevanz).

### Beispiel 1

Zunächst erfolgt die Identifizierung von Kandidaten für Brustkrebs-spezifische Markersequenzen.

In der ersten Phase werden dazu 50 Serumproben von Patientinnen mit Mammakarzinom auf einem MACROarray (umfasst etwa 10.000 unterschiedliche, rekombinante, humane Proteine) untersucht. Dabei werden Kandidaten für Brustkrebs-spezifische Markersequenzen identifiziert.

### Beispiel 2

In der anschließenden Testphase werden diese Kandidaten für Brustkrebs-spezifische Markersequenzen an Serumproben von 100 Brustkrebspatientinnen und 100 Patientinnen mit benignen Veränderungen der Brust bzw. 100 gesunden Kontroll-Patientinnen vergleichend analysiert und charakterisiert. Dadurch diese vergleichende Analyse werden vorwiegend Markersequenzen identifiziert, die mit Brustkrebs-assoziierten Autoantikörpern Wechselwirken.

### Beispiel 3:

Die Auswahl besonders signifikanter Biomarker (Brustkrebs-spezifischer Markersequenzen) erfolgt mittels bioinformatischer Analyse. Die Kandidaten für Brustkrebs-spezifische Markersequenzen werden dahingehend ausgewertet, ob sie zwischen verschiedenen Probanden (z.B. gesund/nicht gesund)/Patientengruppen (z.B. niedriges/hohes Risiko der Metastasenbildung)/Kohorten (z.B. bestimmte Vorgeschichte) diskriminieren.

Dazu werden die Markersequenzkandidaten werden auf einen Biochip aufgebracht und validiert. Die Datenauswertung erfolgt über statistische Analysen, beispielsweise Schwellenwertanalyse, Support Vector Machine Alogrithm (SVM). Der Probenverbrauch für die Validierung beträgt nur 50 µl / Probe. In einem ersten Ansatz werden auf diese Weise Kohorten der Kategorie I und II ausgewählt.

Der erhaltene Biochip ist Brustkrebs spezifisch. Dieser Biochip umfasst ein oder mehrere Brustkrebs-spezifische Markersequenzen und erkennt Brustkrebs-assoziierte Autoantikörper.

Kohorte I: Klinischer Befund Brustkrebs-positive Gruppe (CASE-Gruppe; verifiziert über histopathologischen Befund der Biopsie).

Kohorte II: Klinischer Befund Brustkrebs-negative Gruppe (Kontroll-Gruppe), Alters-gematched.

Patientinnen werden ausgewählt nach Einschluß- und Ausschlusskriterien

Einschlußkriterien
- Histologisch gesichertes Mammakarzinom
- Histologisch gesicherte, nicht-neoplastische Veränderung
- Gesunde altersgematchte Kontrolle
- Zum Zeitpunkt der Diagnose (vor der Operation)
- Vor bzw. während neoadjuvanter und adjuvanter Antiöstrogentherapie, adjuvanter Chemotherapie oder adjuvanter Aromataseinhibitortherapie.

Ausschlusskriterien
- Alter unter 18 Jahren
- Bereits erfolgte Tumorbehandlung

Entsprechende Biochips werden für Diagnose, Voraussage des Therapieverlaufs und Vorhersage der Metastasenbildung entwickelt.

### Beispiel 4:

Für die Entwicklung eines Proteinbiochips für die Diagnose von Brustkrebs, werden die Ergebnisse der Autoantikörperanalyse mit dem goldenen Standard der Diagnose verglichen und die identifizierten Markersequenzen werden validiert (Brustkrebs-spezifische Markersequenzen). Die Ergebnisse werden dann mit anderen klinischen Charakteristika von Brustkrebs, beispielsweise Tumorgröße und Malignität korreliert.

### Beispiel 5:

Bei der Entwicklung eines Proteinbiochips zur Voraussage des Therapieverlaufs wird ein bestimmtes Autoantikörperprofil bzw. ein bestimmtes Signal des Proteinbiochips mit dem Ansprechen des Brustkrebses auf eine bestimmte Therapie korreliert. Darüber hinaus werden Änderungen des Autoantikörperprofils validiert - auch bezüglich verschiedener Behandlungsoptionen (Continuous time modelling).

### Beispiel 6:

Bei der Entwicklung eines Proteinbiochips für die Prädiktion der Metastasenbildung werden Brustkrebs-spezifische Markersequenzen ausgewählt, die mit Brustkrebs-assoziierten Autoantikörpern wechselwirken, die als Indikator für Metastasenbildung geeignet sind. Durch den Vergleich von Autoantikörpern zum Zeitpunkt der Diagnose von Patientinnen mit und ohne Metastasenbildung können Patientinnen identifiziert werden, die ein hohes Metastaserisiko haben.

Im Rahmen der Identifizierung und Validierung von Brustkrebs-spezifischen Markersequenzen können bioinformatische Analysen durchgeführt werden. Für jedes Serum können dazu mittels Microarray Reaktivitäten gegen ca. 2000 unterschiedliche Antigene gemessen werden. Diese Daten werden für ein Ranking der gespotteten Antigene bzgl. ihrer Differenzierungsfähigkeit zwischen gesunden und erkrankten Seren benutzt. Diese Auswertung kann mittels des nicht parametrischen Mann-Whitney Tests auf normalisierten Intensitätsdaten durchgeführt werden. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet.

Desweiteren werden die Daten zur Klassifikation der Seren benutzt. Hierbei kommen unterschiedliche multivariate Methoden zum Einsatz. Dies sind Methoden aus den statistischen Lernverfahren wie Support Vector Machines (SVM), Neuronale Netze oder Klassifikationsbäume, sowie eine Schwellenwertmethode, welche sowohl zur Klassifikation als auch zur visuellen Repräsentation der Daten geeignet ist.

Zur Vermeidung von Overfitting wird beispielsweise eine 10fache Cross-Validierung der Daten durchgeführt.

Die Brustkrebs-spezifischen Markersequenzen (Klonsequenzen (cDNA)) SEQ ID No. 1 - 491 sind dem Sequenzprotokoll zu entnehmen.

**Tabelle A: Daten zu Brustkrebs-spezifischen Markersequenzen (RNA) Klonsequenzen SEQ ID No. 492 - 982**

| SEQ ID No. | Accession No. | Alias Accession No. | Blast |
|---|---|---|---|
| 492 | gi\|261337182 | NM_182905.4 | WAS protein family homolog 1 [Homo sapiens] |
| 493 | gi\|16877437 | BC016965.1 | NLRP1 protein [Homo sapiens] |
| 494 | gi\|57242760 | NM_003804.3 | receptor-interacting serine/threonine-protein kinase 1 [Homo sapiens] |
| 495 | gi\|225579068 | NM_012426.4 | splicing factor 3B subunit 3 [Homo sapiens] |
| 496 | gi\|221136896 | NM_014786.3 | rho guanine nucleotide exchange factor 17 [Homo sapiens] |
| 497 | gi\|60218896 | NM_005748.3 | YY1-associated factor 2 isoform 2 [Homo sapiens] |
| 498 | gi\|157388905 | NM_017806.2 | Ick-interacting transmembrane adapter 1 [Homo sapiens] |
| 499 | gi\|13569955 | NM_030978.1 | actin-related protein 2/3 complex subunit 5-like protein [Homo sapiens] |
| 500 | gi\|17933771 | NM_080388.1 | protein S100-A16 [Homo sapiens] |
| 501 | gi\|171906614 | NM_006891.3 | gamma-crystallin D [Homo sapiens] |
| 502 | gi\|157389013 | NM_001408.2 | cadherin EGF LAG seven-pass G-type receptor 2 precursor [Homo sapiens] |
| 503 | gi\|55956909 | NM_002430.2 | probable tumor suppressor protein MN1 [Homo sapiens] |
| 504 | gi\|21396481 | NM_003512.3 | histone H2A type 1-C [Homo sapiens] |
| 505 | gi\|77812677 | NM_006903.4 | inorganic pyrophosphatase 2, mitochondrial isoform 2 precursor [Homo sapiens] |
| 506 | gi\|41349492 | NM_013239.3 | serine/threonine-protein phosphatase 2A regulatory subunit B" subunit beta [Homo sapiens] |
| 507 | gi\|56118233 | NM_181716.2 | centromere protein V [Homo sapiens] |
| 508 | gi\|225579128 | NM_001018024.2 | mature T-cell proliferation 1 neighbor protein [Homo sapiens] |
| 509 | gi\|334085246 | NM_014889.3 | presequence protease, mitochondrial isoform 2 precursor [Homo sapiens] |
| 510 | gi\|66932989 | NM_001018078.1 | folylpolyglutamate synthase, mitochondrial isoform b [Homo sapiens] |
| 511 | gi\|31317296 | NM_181353.1 | DNA-binding protein inhibitor ID-1 isoform b [Homo sapiens] |
| 512 | gi\|209413724 | NM_003692.3 | tomoregulin-1 precursor [Homo sapiens] |
| 513 | gi\|37181353 | AY358124.1 | SEMA5B [Homo sapiens] |
| 514 | gi\|21704284 | NM_021219.2 | junctional adhesion molecule B precursor [Homo sapiens] |
| 515 | gi\|254028259 | NM_182501.3 | mTERF domain-containing protein 2 [Homo sapiens] |
| 516 | gi\|161016768 | NM_001799.3 | cyclin-dependent kinase 7 [Homo sapiens] |
| 517 | gi\|180636 | M93008.1 | L-isoaspartyl/D-aspartyl protein carboxyl methyltransferase [Homo sapiens] |
| 518 | gi\|67782361 | NM_019030.2 | ATP-dependent RNA helicase DHX29 [Homo sapiens] |
| 519 | gi\|14195613 | NM_018940.2 | protocadherin beta-7 precursor [Homo sapiens] |
| 520 | gi\|93204870 | NM_021229.3 | netrin-4 precursor [Homo sapiens] |
| 521 | gi\|224967099 | NM_001734.3 | complement C1s subcomponent precursor [Homo sapiens] |
| 522 | gi\|16507949 | NM_014466.2 | tektin-2 [Homo sapiens] |
| 523 | gi\|75750475 | NM_001033566.1 | mitochondrial Rho GTPase 1 isoform 2 [Homo sapiens] |
| 524 | gi\|216548626 | NM_001142467.1 | transcription factor HES-4 isoform 1 [Homo sapiens] |
| 525 | gi\|38683798 | NM_033121.1 | ankyrin repeat domain-containing protein 13A [Homo sapiens] |
| 526 | gi\|285002230 | NM_001083112.2 | glycerol-3-phosphate dehydrogenase, mitochondrial precursor [Homo sapiens] |
| 527 | gi\|5454057 | NM_006278.1 | CMP-N-acetylneuraminate-beta-galactosamide-alpha-2,3-sialyltransferase 4 [Homo sapiens] |
| 528 | gi\|169259774 | NM_014737.2 | ras association domain-containing protein 2 [Homo sapiens] |
| 529 | gi\|126091094 | NM_001081563.1 | myotonin-protein kinase isoform 1 [Homo sapiens] |
| 530 | gi\|194097397 | NM_002841.3 | receptor-type tyrosine-protein phosphatase gamma precursor [Homo sapiens] |
| 531 | gi\|41350325 | NM_003791.2 | membrane-bound transcription factor site-1 protease preproprotein [Homo sapiens] |
| 532 | gi\|84508630 | NM_024419.3 | CDP-diacylglycerol--glycerol-3-phosphate 3-phosphatidyltransferase, mitochondrial precursor [Homo sapiens] |
| 533 | gi\|187960072 | NM_006341.3 | mitotic spindle assembly checkpoint protein MAD2B [Homo sapiens] |
| 534 | gi\|76563938 | NM_015920.3 | 40S ribosomal protein S27-like [Homo sapiens] |
| 535 | gi\|169234806 | NM_015401.3 | histone deacetylase 7 isoform a [Homo sapiens] |
| 536 | gi\|120952828 | NM_001079906.1 | zinc finger protein 331 [Homo sapiens] |
| 537 | gi\|57222569 | NM_138477.2 | codanin-1 [Homo sapiens] |
| 538 | gi\|21361096 | NM_004125.2 | DNAJC25-GNG10 protein [Homo sapiens] |
| 539 | gi\|51093843 | NM_004147.3 | developmentally-regulated GTP-binding protein 1 [Homo sapiens] |
| 540 | gi\|221316745 | NM_003156.3 | stromal interaction molecule 1 precursor [Homo sapiens] |
| 541 | gi\|327365362 | NM_203290.2 | DNA-directed RNA polymerases I und III subunit RPAC1 [Homo sapiens] |
| 542 | gi\|114520590 | NM_032985.4 | protein transport protein Sec23B isoform 1 [Homo sapiens] |
| 543 | gi\|194018465 | NM_004454.2 | ETS translocation variant 5 [Homo sapiens] |
| 544 | gi\|62953137 | NM_002192.2 | inhibin beta A chain precursor [Homo sapiens] |
| 545 | gi\|31652260 | NM_002466.2 | myb-related protein B [Homo sapiens] |
| 546 | gi\|85680425 | DQ335454.1 | BS69 variant 3 [Homo sapiens] |
| 547 | gi\|218563704 | NM_025193.3 | 3 beta-hydroxysteroid dehydrogenase type 7 isoform a [Homo sapiens] |
| 548 | gi\|164419759 | NM_000107.2 | DNA damage-binding protein 2 [Homo sapiens] |
| 549 | ggi\|114155138 | NM_153649.3 | tropomyosin alpha-3 chain isoform 2 [Homo sapiens] |
| 550 | gi\|215820618 | NM_014699.3 | zinc finger protein 646 [Homo sapiens] |
| 551 | gi\|157502196 | NM_014738.4 | hypothetical protein LOC9772 [Homo sapiens] |
| 552 | gi\|58331162 | NM_001009936.1 | PHD finger protein 19 isoform b [Homo sapiens] |
| 553 | gi\|110611232 | NM_130445.2 | collagen alpha-1(XVIII) chain isoform 2 precursor [Homo sapiens] |
| 554 | gi\|23110958 | NM_000396.2 | cathepsin K preproprotein [Homo sapiens] |
| 555 | gi\|150417970 | NM_003174.3 | supervillin isoform 1 [Homo sapiens] |
| 556 | gi\|84872172 | NM_003432.1 | zinc finger protein 131 [Homo sapiens] |
| 557 | gi\|54792739 | NM_014858.2 | transmembrane and coiled-coil domains protein 2 isoform 1 [Homo sapiens] |
| 558 | gi\|14589877 | NM_005864.2 | embryonal Fyn-associated substrate isoform 1 [Homo sapiens] |
| 559 | gi\|148529010 | NM_006091.3 | coronin-2B isoform 1 [Homo sapiens] |
| 560 | gi\|51317348 | NM_001003789.1 | rab-like protein 2B isoform 1 [Homo sapiens] |
| 561 | gi\|119120893 | NM_015263.2 | dmX-like protein 2 isoform 2 [Homo sapiens] |
| 562 | gi\|213972618 | NM_001141973.1 | probable cation-transporting ATPase 13A2 isoform 2 [Homo sapiens] |
| 563 | gi\|283135239 | NM_020155.3 | integral membrane protein GPR137 isoform 3 [Homo sapiens] |
| 564 | gi\|164565439 | NM_024571.3 | U11/U12 small nuclear ribonucleoprotein 25 kDa protein [Homo sapiens] |
| 565 | gi\|145331214 | NM_032429.1 | leucine zipper putative tumor suppressor 2 [Homo sapiens] |
| 566 | gi\|94721260 | NM_033133.4 | 2',3'-cyclic-nucleotide 3'-phosphodiesterase [Homo sapiens] |
| 567 | gi\|115511050 | NM_002095.4 | transcription initiation factor IIE subunit beta [Homo sapiens] |
| 568 | gi\|156616274 | NM_002691.2 | DNA polymerase delta catalytic subunit [Homo sapiens] |
| 569 | gi\|38146093 | NM_005481.2 | mediator of RNA polymerase II transcription subunit 16 [Homo sapiens] |
| 570 | gi\|45597176 | NM_015043.3 | TBC1 domain family member 9B isoform b [Homo sapiens] |
| 571 | gi\|23238252 | NM_004377.2 | carnitine O-palmitoyltransferase 1, muscle isoform isoform a [Homo sapiens] |
| 572 | gi\|98986448 | NM_000496.2 | beta-crystallin B2 [Homo sapiens] |
| 573 | gi\|14505784 | NM_000294.1 | phosphorylase b kinase gamma catalytic chain, testis/liver isoform isoform 1 [Homo sapiens] |
| 574 | gi\|78482607 | NM_021129.3 | inorganic pyrophosphatase [Homo sapiens] |
| 575 | gi\|18027315 | AF289556.1 | unknown [Homo sapiens] |
| 576 | gi\|55770885 | NM_006537.2 | ubiquitin carboxyl-terminal hydrolase 3 [Homo sapiens] |
| 577 | gi\|59710102 | NM_006315.4 | polycomb group RING finger protein 3 [Homo sapiens] |
| 578 | gi\|2224666 | AB002361.1 | KIAA0363 [Homo sapiens] |
| 579 | gi\|33356549 | NM_182679.1 | G patch domain-containing protein 4 isoform 2 [Homo sapiens] |
| 580 | gi\|210147473 | NM_022366.2 | dimethyladenosine transferase 2, mitochondrial [Homo sapiens] |
| 581 | gi\|50346003 | NM_002626.4 | 6-phosphofructokinase, liver type [Homo sapiens] |
| 582 | gi\|187828433 | NM_006224.3 | phosphatidylinositol transfer protein alpha isoform [Homo sapiens] |
| 583 | gi\|53749664 | NM_005654.4 | COUP transcription factor 1 [Homo sapiens] |
| 584 | gi\|34485712 | NM_004663.3 | ras-related protein Rab-11A isoform 1 [Homo sapiens] |
| 585 | gi\|150378438 | NM_020226.3 | PR domain zinc finger protein 8 [Homo sapiens] |
| 586 | gi\|52694663 | NM_020799.2 | AMSH-like protease [Homo sapiens] |
| 587 | gi\|169646771 | NM_002064.2 | glutaredoxin-1 [Homo sapiens] |
| 588 | gi\|209969817 | NM_001540.3 | heat shock protein beta-1 [Homo sapiens] |
| 589 | gi\|164664491 | NM_007103.3 | NADH dehydrogenase [ubiquinone] flavoprotein 1, mitochondrial isoform 1 precursor [Homo sapiens] |
| 590 | gi\|48255923 | NM_021075.3 | NADH dehydrogenase [ubiquinone] flavoprotein 3, mitochondrial isoform a precursor [Homo sapiens] |
| 591 | gi\|8923891 | NM_018663.1 | peroxisomal membrane protein 2 [Homo sapiens] |
| 592 | gi\|117553583 | NM_001077523.1 | AP-3 complex subunit delta-1 isoform 1 [Homo sapiens] |
| 593 | gi\|119393885 | NM_014706.3 | squamous cell carcinoma antigen recognized by T-cells 3 [Homo sapiens] |
| 594 | gi\|26787964 | NM_006411.2 | 1-acyl-sn-glycerol-3-phosphate acyltransferase alpha [Homo sapiens] |
| 595 | gi\|10947033 | NM_006454.2 | max dimerization protein 4 [Homo sapiens] |
| 596 | gi\|187960089 | NM_015327.2 | protein SMG5 [Homo sapiens] |
| 597 | gi\|141801721 | NM_015153.2 | PHD finger protein 3 [Homo sapiens] |
| 598 | gi\|187761372 | NM_005744.3 | E3 ubiquitin-protein ligase ARIH1 [Homo sapiens] |
| 599 | gi\|102467483 | NM_017751.2 | sphingomyelin phosphodiesterase 4 isoform 1 [Homo |
| | | | sapiens] |
| 600 | gi\|31543086 | NM_020147.2 | THAP domain-containing protein 10 [Homo sapiens] |
| 601 | gi\|214010239 | NM_001142292.1 | VIP36-like protein isoform 1 [Homo sapiens] |
| 602 | gi\|42476160 | NM_032902.5 | protein phosphatase 1 regulatory subunit 16A [Homo sapiens] |
| 603 | gi\|333609250 | NM_001005850.2 | zinc finger protein 835 [Homo sapiens] |
| 604 | gi\|189011564 | NM_000038.4 | adenomatous polyposis coli protein isoform b [Homo sapiens] |
| 605 | gi\|87578391 | NM_031845.2 | microtubule-associated protein 2 isoform 2 [Homo sapiens] |
| 606 | gi\|19913344 | NM_006527.2 | histone RNA hairpin-binding protein [Homo sapiens] |
| 607 | gi\|110225356 | NM_006819.2 | stress-induced-phosphoprotein 1 [Homo sapiens] |
| 608 | gi\|42544225 | NM_020857.2 | vacuolar protein sorting-associated protein 18 homolog [Homo sapiens] |
| 609 | gi\|166795298 | NM_006516.2 | solute carrier family 2, facilitated glucose transporter member 1 [Homo sapiens] |
| 610 | gi\|211904132 | NM_014764.3 | DAZ-associated protein 2 isoform a [Homo sapiens] |
| 611 | gi\|148612828 | NM_001098509.1 | small G protein signaling modulator 2 isoform 2 [Homo sapiens] |
| 612 | gi\|40018634 | NM_015440.3 | monofunctional C1-tetrahydrofolate synthase, mitochondrial isoform 2 precursor [Homo sapiens] |
| 613 | gi\|169234785 | NM_017991.4 | hypothetical protein LOC55683 isoform b [Homo sapiens] |
| 614 | gi\|70778868 | NM_021933.2 | migration and invasion-inhibitory protein [Homo sapiens] |
| 615 | gi\|113204623 | NM_032193.3 | ribonuclease H2 subunit C [Homo sapiens] |
| 616 | gi\|65301131 | NM_138338.2 | DNA-directed RNA polymerase III subunit RPC8 isoform a [Homo sapiens] |
| 617 | gi\|221219069 | NM_145295.3 | zinc finger protein 627 [Homo sapiens] |
| 618 | gi\|21264342 | NM_002967.2 | scaffold attachment factor B1 isoform 3 [Homo sapiens] |
| 619 | gi\|197085592 | NM_006328.3 | RNA-binding protein 14 isoform 1 [Homo sapiens] |
| 620 | gi\|149588533 | NM_001098833.1 | ataxin-7-like protein 3 isoform b [Homo sapiens] |
| 621 | gi\|20270356 | NM_138802.1 | AN1-type zinc finger protein 2B [Homo sapiens] |
| 622 | gi\|35493700 | NM_152564.3 | vacuolar protein sorting-associated protein 13B isoform 1 [Homo sapiens] |
| 623 | gill42385096 | NM_001077693.2 | endothelial cell-specific chemotaxis regulator precursor [Homo sapiens] |
| 624 | gi\|71773207 | NM_000683.3 | alpha-2C adrenergic receptor [Homo sapiens] |
| 625 | gi\|55925575 | NM_000597.2 | insulin-like growth factor-binding protein 2 precursor [Homo sapiens] |
| 626 | gi\|71772582 | NM_001030001.1 | 40S ribosomal protein S29 isoform 2 [Homo sapiens] |
| 627 | gi\|18105053 | NM_014970.2 | kinesin-associated protein 3 isoform 1 [Homo sapiens] |
| 628 | gi\|34147578 | NM_014338.3 | phosphatidylserine decarboxylase proenzyme [Homo sapiens] |
| 629 | gi\|12060856 | AF308303.1 | serologically defined breast cancer antigen NY-BR-99 [Homo sapiens] |
| 630 | gi\|7020624 | AK000496.1 | unnamed protein product [Homo sapiens] |
| 631 | gi\|209571529 | NM_182527.2 | calcium-binding protein 7 [Homo sapiens] |
| 632 | gi\|56090145 | NM_001005920.2 | jmjC domain-containing protein 8 [Homo sapiens] |
| 633 | gi\|166235894 | NM_001114089.1 | ectonucleoside triphosphate diphosphohydrolase 6 isoform 2 [Homo sapiens] |
| 634 | gi\|189571686 | NM_003731.2 | Sjoegren syndrome nuclear autoantigen 1 [Homo sapiens] |
| 635 | gi\|156071530 | NM_145802.3 | septin-6 isoform D [Homo sapiens] |
| 636 | gi\|98961132 | NM_025112.4 | zinc finger protein ZXDC isoform 1 [Homo sapiens] |
| 637 | gi\|66267729 | NM_052844.3 | WD repeat-containing protein 34 [Homo sapiens] |
| 638 | gi\|224586869 | NM_138443.3 | HAUS augmin-like complex subunit 1 [Homo sapiens] |
| 639 | gi\|197276595 | NM_006129.3 | bone morphogenetic protein 1 isoform 3 precursor [Homo sapiens] |
| 640 | gi\|161727457 | AB370195.1 | dihydropyrimidinase-like 2 long form [Homo sapiens] |
| 641 | gi\|89276761 | NM_032940.2 | DNA-directed RNA polymerase II subunit RPB3 [Homo sapiens] |
| 642 | gi\|197209876 | NM_003496.2 | transformation/transcription domain-associated protein [Homo sapiens] |
| 643 | gi\|119393884 | NM_005146.4 | U4/U6.U5 tri-snRNP-associated protein 1 [Homo sapiens] |
| 644 | gi\|38201691 | NM_007368.2 | ras GTPase-activating protein 3 [Homo sapiens] |
| 645 | gi\|22091458 | NM_014303.2 | pescadillo homolog [Homo sapiens] |
| 646 | gi\|55774983 | NM_015660.2 | GTPase IMAP family member 2 [Homo sapiens] |
| 647 | gi\|118498363 | NM_001079520.1 | dapper homolog 1 isoform 2 [Homo sapiens] |
| 648 | gi\|85062625 | NM_032377.3 | transcription elongation factor 1 homolog [Homo sapiens] |
| 649 | gi\|20357526 | NM_002074.2 | guanine nucleotide-binding protein G(l)/G(S)/G(T) subunit beta-1 [Homo sapiens] |
| 650 | gi\|194363754 | NM_000852.3 | glutathione S-transferase P [Homo sapiens] |
| 651 | gi\|46255046 | NM_001535.2 | protein arginine N-methyltransferase 2 isoform 1 [Homo sapiens] |
| 652 | gi\|157412269 | NM_031203.2 | heterogeneous nuclear ribonucleoprotein M isoform b [Homo sapiens] |
| 653 | gi\|223029468 | NM_006159.2 | protein kinase C-binding protein NELL2 isoform b precursor [Homo sapiens] |
| 654 | gi\|57863258 | NM_001008897.1 | T-complex protein 1 subunit alpha isoform b [Homo sapiens] |
| 655 | gi\|147078256 | NM_003778.3 | beta-1,4-galactosyltransferase 4 [Homo sapiens] |
| 656 | gi\|145611425 | NM_006814.3 | proteasome inhibitor P131 subunit [Homo sapiens] |
| 657 | gi\|141406095 | NM_014003.3 | pre-mRNA-splicing factor ATP-dependent RNA helicase PRP16 [Homo sapiens] |
| 658 | gi\|178557737 | NM_006371.4 | cartilage-associated protein precursor [Homo sapiens] |
| 659 | gi\|91208424 | NM_012469.3 | pre-mRNA-processing factor 6 [Homo sapiens] |
| 660 | gi\|51871119 | NM_138401.2 | multivesicular body subunit 12A [Homo sapiens] |
| 661 | gi\|260898772 | NM_176812.4 | charged multivesicular body protein 4b [Homo sapiens] |
| 662 | gi\|142388930 | NM_152732.3 | radial spoke head protein 9 homolog isoform 1 [Homo sapiens] |
| 663 | gi\|320089575 | NM_001004333.4 | ribonuclease kappa [Homo sapiens] |
| 664 | gi\|76496473 | NM_000018.2 | very long-chain specific acyl-CoA dehydrogenase, mitochondrial isoform 1 precursor [Homo sapiens] |
| 665 | gi\|21618333 | NM_004003.2 | carnitine acetyltransferase isoform 2 [Homo sapiens] |
| 666 | gi\|38201713 | NM_001419.2 | ELAV-like protein 1 [Homo sapiens] |
| 667 | gi\|296531407 | NM_198155.3 | ES1 protein homolog, mitochondrial isoform Ib precursor [Homo sapiens] |
| 668 | gi\|45269143 | NM_012289.3 | kelch-like ECH-associated protein 1 [Homo sapiens] |
| 669 | gi\|41872645 | NM_014780.3 | cullin-7 isoform 2 [Homo sapiens] |
| 670 | gi\|42544178 | NM_012127.2 | cipl-interacting zinc finger protein isoform 1 [Homo sapiens] |
| 671 | gi\|34147607 | NM_015953.3 | nitric oxide synthase-interacting protein [Homo sapiens] |
| 672 | gi\|124256477 | NM_017974.3 | autophagy-related protein 16-1 isoform 2 [Homo sapiens] |
| 673 | gi\|110349768 | NM_024296.3 | coiled-coil domain-containing protein 28B [Homo sapiens] |
| 674 | gi\|40555764 | BC064481.1 | RNF187 protein [Homo sapiens] |
| 675 | gi\|194385887 | AK304513.1 | unnamed protein product [Homo sapiens] |
| 676 | gi\|194328721 | NM_001045556.2 | src-like-adapter isoform a [Homo sapiens] |
| 677 | gi\|31652219 | NM_133640.3 | mediator of RNA polymerase II transcription subunit 22 isoform b [Homo sapiens] |
| 678 | gi\|221136767 | NM_000371.3 | transthyretin precursor [Homo sapiens] |
| 679 | gi\|36287059 | NM_182709.1 | histone acetyltransferase KAT5 isoform 3 [Homo sapiens] |
| 680 | gi\|261399873 | NM_001009570.2 | T-complex protein 1 subunit eta isoform b [Homo sapiens] |
| 681 | gi\|42544141 | NM_202494.1 | PDZ domain-containing protein GIPC1 isoform 2 [Homo sapiens] |
| 682 | gi\|88501739 | NM_007032.5 | TRIO and F-actin-binding protein isoform 1 [Homo sapiens] |
| 683 | gi\|144953896 | NM_007184.3 | nischarin [Homo sapiens] |
| 684 | gi\|58331180 | NM_134265.2 | WD repeat and SOCS box-containing protein 1 isoform 2 [Homo sapiens] |
| 685 | gi\|217272837 | NM_006623.3 | D-3-phosphoglycerate dehydrogenase [Homo sapiens] |
| 686 | gi\|221136945 | NM_014502.4 | pre-mRNA-processing factor 19 [Homo sapiens] |
| 687 | gi\|117553614 | NM_016045.2 | protein slowmo homolog 2 [Homo sapiens] |
| 688 | gi\|15967154 | NM_016558.2 | SCAN domain-containing protein 1 isoform 1 [Homo sapiens] |
| 689 | gi\|74048433 | NM_016309.2 | leucine carboxyl methyltransferase 1 isoform a [Homo sapiens] |
| 690 | gi\|8922734 | NM_018255.1 | elongator complex protein 2 isoform 2 [Homo sapiens] |
| 691 | gi\|145275199 | NM_025080.3 | L-asparaginase [Homo sapiens] |
| 692 | gi\|42734378 | NM_138350.2 | THAP domain-containing protein 3 isoform 2 [Homo sapiens] |
| 693 | gi\|209915551 | NM_001760.3 | G1/S-specific cyclin-D3 isoform 2 [Homo sapiens] |
| 694 | gi\|156071497 | NM_004952.4 | ephrin-A3 precursor [Homo sapiens] |
| 695 | gi\|225579056 | NM_003049.3 | sodium/bile acid cotransporter [Homo sapiens] |
| 696 | gi\|284005308 | NM_004260.3 | ATP-dependent DNA helicase Q4 [Homo sapiens] |
| 697 | gi\|50726974 | NM_138501.4 | trans-2,3-enoyl-CoA reductase [Homo sapiens] |
| 698 | gi\|59710114 | NM_014679.3 | centrosomal protein of 57 kDa [Homo sapiens] |
| 699 | gi\|45643118 | NM_006117.2 | enoyl-CoA delta isomerase 2, mitochondrial isoform 1 [Homo sapiens] |
| 700 | gi\|111120323 | NM_006621.4 | putative adenosylhomocysteinase 2 isoform a [Homo sapiens] |
| 701 | gi\|33469977 | NM_182835.1 | sec1 family domain-containing protein 1 isoform b [Homo sapiens] |
| 702 | gi\|118200355 | NM_014394.2 | growth hormone-inducible transmembrane protein [Homo sapiens] |
| 703 | gi\|59850648 | NM_018443.2 | zinc finger protein 302 [Homo sapiens] |
| 704 | gi\|142371393 | NM_181505.2 | protein phosphatase 1 regulatory subunit 1B isoform 2 [Homo sapiens] |
| 705 | gi\|168480109 | NM_005225.2 | transcription factor E2F1 [Homo sapiens] |
| 706 | gi\|51102290 | NM_005395.2 | postmeiotic segregation increased 2-like 3 isoform 1 [Homo sapiens] |
| 707 | gi\|48255969 | NM_002969.3 | mitogen-activated protein kinase 12 [Homo sapiens] |
| 708 | gi\|170650722 | NM_014236.3 | dihydroxyacetone phosphate acyltransferase [Homo sapiens] |
| 709 | gi\|148527950 | NM_013365.3 | ADP-ribosylation factor-binding protein GGA1 isoform 1 [Homo sapiens] |
| 710 | gi\|213385322 | NM_001137559.1 | anaphase-promoting complex subunit 5 isoform b [Homo sapiens] |
| 711 | gi\|118498335 | NM_016520.2 | chromosome 9 open reading frame 78 [Homo sapiens] |
| 712 | gi\|37537686 | NM_018337.2 | zinc finger protein 444 [Homo sapiens] |
| 713 | gi\|68533248 | NM_018476.3 | protein BEX1 [Homo sapiens] |
| 714 | gi\|192449448 | NM_022066.3 | ubiquitin-conjugating enzyme E2 O [Homo sapiens] |
| 715 | gi\|12232384 | NM_022730.1 | COP9 signalosome complex subunit 7b [Homo sapiens] |
| 716 | gi\|201025398 | NM_024334.2 | transmembrane protein 43 [Homo sapiens] |
| 717 | gi\|38016923 | NM_006253.4 | 5'-AMP-activated protein kinase subunit beta-1 [Homo sapiens] |
| 718 | gi\|77404354 | NM_003908.3 | eukaryotic translation initiation factor 2 subunit 2 [Homo sapiens] |
| 719 | gi\|237649014 | NM_006824.2 | probable rRNA-processing protein EBP2 isoform 2 [Homo sapiens] |
| 720 | gi\|40068460 | NM_007284.3 | twinfilin-2 [Homo sapiens] |
| 721 | gi\|39780570 | NM_018116.2 | protein misato homolog 1 [Homo sapiens] |
| 722 | gi\|40068484 | NM_022834.3 | von Willebrand factor A domain-containing protein 1 isoform 1 precursor [Homo sapiens] |
| 723 | gi\|217416378 | NM_001142650.1 | heterogeneous nuclear ribonucleoprotein L-like isoform 2 [Homo sapiens] |
| 724 | gi\|71834871 | NM_178865.3 | serine incorporator 2 isoform 1 [Homo sapiens] |
| 725 | gi\|258547122 | NM_001151.3 | ADP/ATP translocase 1 [Homo sapiens] |
| 726 | gi\|260593722 | NM_001983.3 | DNA excision repair protein ERCC-1 isoform 2 [Homo sapiens] |
| 727 | gi\|52630340 | NM_002107.3 | histone H3.3 [Homo sapiens] |
| 728 | gi\|219879807 | NM_006325.3 | GTP-binding nuclear protein Ran [Homo sapiens] |
| 729 | gi\|23111017 | NM_152856.1 | RNA-binding protein 10 isoform 2 [Homo sapiens] |
| 730 | gi\|157785644 | NM_005876.4 | striated muscle preferentially expressed protein kinase isoform 1 [Homo sapiens] |
| 731 | gi\|52851442 | NM_006360.3 | eukaryotic translation initiation factor 3 subunit M [Homo sapiens] |
| 732 | gi\|197100772 | NM_020320.3 | probable arginyl-tRNA synthetase, mitochondrial precursor [Homo sapiens] |
| 733 | gi\|89337269 | NM_022749.5 | retinoic acid induced 16 [Homo sapiens] |
| 734 | gi\|47174858 | NM_025058.3 | tripartite motif-containing protein 46 [Homo sapiens] |
| 735 | gi\|109638742 | NM_031478.4 | hypothetical protein LOC83723 [Homo sapiens] |
| 736 | gi\|53729358 | NM_138361.3 | E3 ubiquitin-protein ligase LRSAM1 isoform 1 [Homo sapiens] |
| 737 | gi\|4506026 | NM_002720.1 | serine/threonine-protein phosphatase 4 catalytic subunit [Homo sapiens] |
| 738 | gi\|73858576 | NM_003254.2 | metalloproteinase inhibitor 1 precursor [Homo sapiens] |
| 739 | gi\|109150415 | NM_012293.1 | peroxidasin homolog precursor [Homo sapiens] |
| 740 | gi\|34365370 | BX641004.1 | hypothetical protein [Homo sapiens] |
| 741 | gi\|6005793 | NM_007213.1 | PRA1 family protein 2 [Homo sapiens] |
| 742 | gi\|33859677 | NM_017879.1 | zinc finger protein 416 [Homo sapiens] |
| 743 | gi\|149274652 | NM_020780.1 | patched domain-containing protein 2 [Homo sapiens] |
| 744 | gi\|50511940 | NM_133455.2 | EMI domain-containing protein 1 [Homo sapiens] |
| 745 | gi\|187608297 | NM_001660.3 | ADP-ribosylation factor 4 [Homo sapiens] |
| 746 | gi\|34335193 | NM_182720.1 | cAMP-responsive element modulator isoform g [Homo sapiens] |
| 747 | gi\|40549399 | NM_001894.4 | casein kinase I isoform epsilon [Homo sapiens] |
| 748 | gi\|197245333 | NM_005273.3 | guanine nucleotide-binding protein G(I)/G(S)/G(T) subunit beta-2 [Homo sapiens] |
| 749 | gi\|188497749 | NM_033500.2 | hexokinase-1 isoform HKI-td [Homo sapiens] |
| 750 | gi\|187828416 | NM_005586.3 | myoD family inhibitor [Homo sapiens] |
| 751 | gi\|38679891 | NM_006223.2 | peptidyl-prolyl cis-trans isomerase NIMA-interacting 4 isoform 1 [Homo sapiens] |
| 752 | gi\|221218992 | NM_003475.3 | ras association domain-containing protein 7 isoform 1 [Homo sapiens] |
| 753 | gi\|156071485 | NM_003680.3 | tyrosyl-tRNA synthetase, cytoplasmic [Homo sapiens] |
| 754 | gi\|58530880 | NM_004675.2 | GTP-binding protein Di-Ras3 [Homo sapiens] |
| 755 | gi\|150170698 | NM_015656.1 | kinesin-like protein K1F26A [Homo sapiens] |
| 756 | gi\|198386318 | NM_016188.4 | actin-like protein 6B [Homo sapiens] |
| 757 | gi\|49574501 | NM_016243.2 | NADH-cytochrome b5 reductase 1 [Homo sapiens] |
| 758 | gi\|34147350 | NM_023940.2 | ras-like protein family member 11B [Homo sapiens] |
| 759 | gi\|55749599 | NM_032451.1 | protein spire homolog 2 [Homo sapiens] |
| 760 | gi\|39992615 | BC064477.1 | PIM3 protein [Homo sapiens] |
| 761 | gi\|56118216 | NM_001008216.1 | UDP-glucose 4-epimerase [Homo sapiens] |
| 762 | gi\|27502382 | NM_172316.1 | homeobox protein Meis2 isoform h [Homo sapiens] |
| 763 | gi\|91208422 | NM_003302.2 | thyroid receptor-interacting protein 6 [Homo sapiens] |
| 764 | gi\|154800448 | NM_003433.3 | zinc finger protein 132 [Homo sapiens] |
| 765 | gi\|41872688 | NM_003660.2 | liprin-alpha-3 [Homo sapiens] |
| 766 | gi\|197382802 | NM_006769.3 | LIM domain transcription factor LMO4 [Homo sapiens] |
| 767 | gi\|253735774 | NM_001162383.1 | rho guanine nucleotide exchange factor 2 isoform 1 [Homo sapiens] |
| 768 | gi\|5730026 | NM_006559.1 | KH domain-containing, RNA-binding, signal transduction-associated protein 1 [Homo sapiens] |
| 769 | gi\|219555664 | NM_001143780.1 | solute carrier family 25 member 39 isoform a [Homo sapiens] |
| 770 | gi\|41393557 | NM_018032.3 | putative RNA-binding protein Luc7-like 1 isoform a [Homo sapiens] |
| 771 | gi\|149274623 | NM_030915.3 | protein LBH [Homo sapiens] |
| 772 | gi\|58761547 | NM_032538.1 | tau-tubulin kinase 1 [Homo sapiens] |
| 773 | gi\|34147461 | NM_033414.2 | zinc finger protein 622 [Homo sapiens] |
| 774 | gi\|304555613 | NM_152743.3 | BRCA1-associated ATM activator 1 [Homo sapiens] |
| 775 | gi\|229608893 | NM_181723.2 | EF-hand domain-containing family member A2 [Homo sapiens] |
| 776 | gi\|119964727 | NM_152783.3 | D-2-hydroxyglutarate dehydrogenase, mitochondrial precursor [Homo sapiens] |
| 777 | gi\|109148541 | NM_001605.2 | alanyl-tRNA synthetase, cytoplasmic [Homo sapiens] |
| 778 | gi\|83700234 | NM_001967.3 | eukaryotic initiation factor 4A-II [Homo sapiens] |
| 779 | gi\|105990523 | NM_000182.4 | trifunctional enzyme subunit alpha, mitochondrial precursor [Homo sapiens] |
| 780 | gi\|18201902 | NM_002109.3 | histidyl-tRNA synthetase, cytoplasmic [Homo sapiens] |
| 781 | gi\|78190460 | NM_000984.5 | 60S ribosomal protein L23a [Homo sapiens] |
| 782 | gi\|78190459 | NM_000978.3 | 60S ribosomal protein L23 [Homo sapiens] |
| 783 | gi\|45446742 | NM_007372.2 | ATP-dependent RNA helicase DDX42 [Homo sapiens] |
| 784 | gi\|61102726 | NM_015315.3 | la-related protein 1 isoform 1 [Homo sapiens] |
| 785 | gi\|112382376 | NM_014501.2 | ubiquitin-conjugating enzyme E2 S [Homo sapiens] |
| 786 | gi\|82830423 | NM_001037533.1 | GON-4-like protein isoform a [Homo sapiens] |
| 787 | gi\|110227844 | NM_024112.3 | hypothetical protein LOC79095 [Homo sapiens] |
| 788 | gi\|149363677 | NM_199287.2 | coiled-coil domain-containing protein 137 [Homo sapiens] |
| 789 | gi\|194353969 | NM_000681.3 | alpha-2A adrenergic receptor [Homo sapiens] |
| 790 | gi\|11038670 | NM_004339.2 | pituitary tumor-transforming gene 1 protein-interacting protein precursor [Homo sapiens] |
| 791 | gi\|87159810 | NM_004357.4 | CD151 antigen [Homo sapiens] |
| 792 | gi\|166235161 | NM_001839.3 | calponin-3 [Homo sapiens] |
| 793 | gi\|46411186 | NM_005234.3 | nuclear receptor subfamily 2 group F member 6 [Homo sapiens] |
| 794 | gi\|156071503 | NM_006908.4 | ras-related C3 botulinum toxin substrate 1 isoform Rac1 [Homo sapiens] |
| 795 | gi\|37577134 | NM_003348.3 | ubiquitin-conjugating enzyme E2 N [Homo sapiens] |
| 796 | gi\|37594440 | NM_003437.2 | zinc finger protein 136 [Homo sapiens] |
| 797 | gi\|197333754 | NM_006764.4 | interferon-related developmental regulator 2 [Homo sapiens] |
| 798 | gi\|31652256 | NM_005461.3 | transcription factor MafB [Homo sapiens] |
| 799 | gi\|114796625 | NM_013356.2 | monocarboxylate transporter 3 [Homo sapiens] |
| 800 | gi\|18496982 | NM_015526.1 | CAP-Gly domain-containing linker protein 3 [Homo sapiens] |
| 801 | gi\|323635445 | NM_020394.4 | zinc finger protein 695 isoform 1 [Homo sapiens] |
| 802 | gi\|197245455 | NM_022574.4 | PERQ amino acid-rich with GYF domain-containing protein 1 [Homo sapiens] |
| 803 | gi\|186928846 | NM_032830.2 | cirhin [Homo sapiens] |
| 804 | gi\|62241010 | NM_005163.2 | RAC-alpha serine/threonine-protein kinase [Homo sapiens] |
| 805 | gi\|38372939 | NM_001185.2 | zinc-alpha-2-glycoprotein precursor [Homo sapiens] |
| 806 | gi\|21735620 | NM_005918.2 | malate dehydrogenase, mitochondrial precursor [Homo sapiens] |
| 807 | gi\|115387093 | NM_003000.2 | succinate dehydrogenase [ubiquinone] iron-sulfur subunit, mitochondrial precursor [Homo sapiens] |
| 808 | gi\|54607088 | NM_001005914.1 | semaphorin-3B isoform 2 precursor [Homo sapiens] |
| 809 | gi\|56550050 | NM_006590.2 | U4/U6.U5 tri-snRNP-associated protein 2 [Homo sapiens] |
| 810 | gi\|38027945 | NM_006833.4 | COP9 signalosome complex subunit 6 [Homo sapiens] |
| 811 | gi\|57617038 | NM_015140.2 | tubulin--tyrosine ligase-like protein 12 [Homo sapiens] |
| 812 | gi\|110227859 | NM_016305.2 | SS18-like protein 2 [Homo sapiens] |
| 813 | gi\|222831567 | NM_033082.3 | SAP domain-containing ribonucleoprotein [Homo sapiens] |
| 814 | gi\|40548381 | NM_199249.1 | multidrug resistance-related protein [Homo sapiens] |
| 815 | gi\|89886471 | NM_206538.2 | hematopoietic signal peptide-containing isoform 2 [Homo sapiens] |
| 816 | gi\|19913440 | NM_002149.2 | hippocalcin-like protein 1 [Homo sapiens] |
| 817 | gi\|316983123 | NM_021029.5 | 60S ribosomal protein L36a isoform a [Homo sapiens] |
| 818 | gi\|55925657 | NM_002997.4 | syndecan-1 precursor [Homo sapiens] |
| 819 | gi\|46877103 | NM_003367.2 | upstream stimulatory factor 2 isoform 1 [Homo sapiens] |
| 820 | gi\|88853068 | NM_000638.3 | vitronectin precursor [Homo sapiens] |
| 821 | gi\|4503824 | NM_003505.1 | frizzled-1 precursor [Homo sapiens] |
| 822 | gi\|133908634 | NM_012109.2 | transmembrane protein 59-like precursor [Homo sapiens] |
| 823 | gi\|151101234 | NM_012461.2 | TERF1-interacting nuclear factor 2 isoform 2 [Homo sapiens] |
| 824 | gi\|221316691 | NM_020309.3 | vesicular glutamate transporter 1 [Homo sapiens] |
| 825 | gi\|165932369 | NM_024582.4 | protocadherin Fat 4 precursor [Homo sapiens] |
| 826 | gi\|52353305 | NM_001005210.1 | leucine-rich repeat-containing protein 55 [Homo sapiens] |
| 827 | gi\|194378373 | AK294825.1 | unnamed protein product [Homo sapiens] |
| 828 | gi\|32454740 | NM_001235.2 | serpin H1 precursor [Homo sapiens] |
| 829 | gi\|33598925 | NM_005506.2 | lysosome membrane protein 2 isoform 1 [Homo sapiens] |
| 830 | gi\|18201904 | NM_000175.2 | glucose-6-phosphate isomerase isoform 2 [Homo sapiens] |
| 831 | gi\|161169039 | NM_004548.2 | NADH dehydrogenase [ubiquinone] 1 beta subcomplex subunit 10 [Homo sapiens] |
| 832 | gi\|141801911 | NM_003295.2 | translationally-controlled tumor protein [Homo sapiens] |
| 833 | gi\|23397695 | NM_152925.1 | copine-1 isoform a [Homo sapiens] |
| 834 | gi\|197313723 | NM_005736.3 | alpha-centractin [Homo sapiens] |
| 835 | gi\|34147665 | NM_006374.3 | serine/threonine-protein kinase 25 [Homo sapiens] |
| 836 | gi\|54112115 | NM_006802.2 | splicing factor 3A subunit 3 [Homo sapiens] |
| 837 | gi\|63082031 | NM_015089.2 | cullin-9 [Homo sapiens] |
| 838 | gi\|41872442 | NM_199368.1 | short transient receptor potential channel 4-associated protein isoform b [Homo sapiens] |
| 839 | gi\|197100212 | NM_001610.2 | lysosomal acid phosphatase isoform 1 precursor [Homo sapiens] |
| 840 | gi\|169636438 | NM_000078.2 | cholesteryl ester transfer protein precursor [Homo sapiens] |
| 841 | gi\|169259765 | NM_001514.5 | transcription initiation factor IIB [Homo sapiens] |
| 842 | gi\|63253297 | NM_003132.2 | spermidine synthase [Homo sapiens] |
| 843 | gi\|37577149 | NM_016453.2 | NCK-interacting protein with SH3 domain isoform 1 [Homo sapiens] |
| 844 | gi\|37622893 | NM_194460.1 | RING finger protein 126 [Homo sapiens] |
| 845 | gi\|29171685 | NM_030768.2 | integrin-linked kinase-associated serine/threonine phosphatase 2C [Homo sapiens] |
| 846 | gi\|14150140 | NM_032346.1 | programmed cell death protein 2-like [Homo sapiens] |
| 847 | gi\|119120876 | NM_133474.2 | zinc finger protein 721 [Homo sapiens] |
| 848 | gi\|257471022 | NM_004930.3 | F-actin-capping protein subunit beta isoform 1 [Homo sapiens] |
| 849 | gi\|19718776 | NM_004111.4 | flap endonuclease 1 [Homo sapiens] |
| 850 | gi\|24797084 | NM_002265.4 | importin subunit beta-1 [Homo sapiens] |
| 851 | gi\|221316755 | NM_000425.3 | neural cell adhesion molecule L1 isoform 1 precursor [Homo sapiens] |
| 852 | gi\|171543862 | NM_000289.4 | 6-phosphofructokinase, muscle type isoform 2 [Homo sapiens] |
| 853 | gi\|4505940 | NM_000938.1 | DNA-directed RNA polymerase II subunit RPB2 [Homo sapiens] |
| 854 | gi\|156631004 | NM_002812.4 | 26S proteasome non-ATPase regulatory subunit 8 [Homo sapiens] |
| 855 | gi\|51477705 | NM_003078.3 | SWI/SNF-related matrix-associated actin-dependent regulator of chromatin subfamily D member 3 isoform 1 [Homo sapiens] |
| 856 | gi\|38505154 | NM_003195.4 | transcription elongation factor A protein 2 isoform a [Homo sapiens] |
| 857 | gi\|63162571 | NM_005998.3 | T-complex protein 1 subunit gamma isoform a [Homo sapiens] |
| 858 | gi\|55769586 | NM_003703.1 | nucleolar protein 14 [Homo sapiens] |
| 859 | gi\|95147537 | NM_006051.3 | amyloid beta A4 precursor protein-binding family B member 3 isoform d [Homo sapiens] |
| 860 | gi\|8922332 | NM_018049.1 | pleckstrin homology domain-containing family J member 1 [Homo sapiens] |
| 861 | gi\|32484989 | NM_018639.3 | WD repeat and SOCS box-containing protein 2 [Homo sapiens] |
| 862 | gi\|215599267 | NM_032039.2 | protein ITFG3 [Homo sapiens] |
| 863 | gi\|21362049 | NM_032357.2 | coiled-coil domain-containing protein 115 [Homo sapiens] |
| 864 | gi\|160420327 | NM_194279.2 | iron-sulfur cluster assembly 2 homolog, mitochondrial |
| | | | precursor [Homo sapiens] |
| 865 | gi\|11494227 | NM_001418.3 | eukaryotic translation initiation factor 4 gamma 2 |
| | | | isoform 1 [Homo sapiens] |
| 866 | gi\|61835203 | NM_001013436.1 | 3-mercaptopyruvate sulfurtransferase isoform 2 [Homo sapiens] |
| 867 | gi\|39725687 | NM_005002.3 | NADH dehydrogenase [ubiquinone] 1 alpha subcomplex subunit 9, mitochondrial precursor [Homo sapiens] |
| 868 | gi\|106049291 | NM_022172.2 | pyruvate carboxylase, mitochondrial precursor [Homo sapiens] |
| 869 | gi\|156416002 | NM_004168.2 | succinate dehydrogenase [ubiquinone] flavoprotein subunit, mitochondrial precursor [Homo sapiens] |
| 870 | gi\|209413761 | NM_017586.2 | calcium channel flower homolog isoform a [Homo sapiens] |
| 871 | gi\|166197689 | NM_001114090.1 | ephexin-1 isoform 2 [Homo sapiens] |
| 872 | gi\|75677342 | NM_015544.2 | transmembrane protein 98 [Homo sapiens] |
| 873 | gi\|187829451 | NM_001127231.1 | autism susceptibility gene 2 protein isoform 2 [Homo sapiens] |
| 874 | gi\|218751872 | NM_018645.4 | transcription cofactor HES-6 isoform a [Homo sapiens] |
| 875 | gi\|91807118 | NM_025141.3 | TM2 domain-containing protein 3 isoform b [Homo sapiens] |
| 876 | gi\|114520614 | NM_001954.4 | epithelial discoidin domain-containing receptor 1 isoform 1 precursor [Homo sapiens] |
| 877 | gi\|186659502 | NM_031263.2 | heterogeneous nuclear ribonucleoprotein K isoform a [Homo sapiens] |
| 878 | gi\|313760623 | NM_000442.4 | platelet endothelial cell adhesion molecule precursor [Homo sapiens] |
| 879 | gi\|34335279 | NM_002811.3 | 26S proteasome non-ATPase regulatory subunit 7 [Homo sapiens] |
| 880 | gi\|30581139 | NM_006263.2 | proteasome activator complex subunit 1 isoform 1 [Homo sapiens] |
| 881 | gi\|78191800 | NM_000997.4 | 60S ribosomal protein L37 [Homo sapiens] |
| 882 | gi\|197927095 | NM_001030.4 | 40S ribosomal protein S27 [Homo sapiens] |
| 883 | gi\|21396499 | NM_003609.2 | HIRA-interacting protein 3 [Homo sapiens] |
| 884 | gi\|25777595 | NM_003648.2 | diacylglycerol kinase delta isoform 1 [Homo sapiens] |
| 885 | gi\|52486264 | NM_006029.4 | paraneoplastic antigen Ma1 [Homo sapiens] |
| 886 | gi\|57242773 | NM_014680.2 | hypothetical protein LOC9703 precursor [Homo sapiens] |
| 887 | gi\|151108508 | NM_014859.4 | rho GTPase-activating protein 44 [Homo sapiens] |
| 888 | gi\|104876422 | NM_006040.2 | heparan sulfate glucosamine 3-O-sulfotransferase 4 [Homo sapiens] |
| 889 | gi\|110349723 | NM_021267.3 | LAG1 longevity assurance homolog 1 isoform 1 [Homo sapiens] |
| 890 | gi\|166795249 | NM_006845.3 | kinesin-like protein KIF2C [Homo sapiens] |
| 891 | gi\|38570153 | NM_012279.2 | zinc finger protein 346 [Homo sapiens] |
| 892 | gi\|156602659 | NM_018206.4 | vacuolar protein sorting-associated protein 35 [Homo sapiens] |
| 893 | gi\|17978480 | NM_080413.1 | vacuolar protein sorting-associated protein 16 homolog isoform 3 [Homo sapiens] |
| 894 | gi\|40807483 | NM_030815.2 | p53 and DNA damage-regulated protein 1 [Homo sapiens] |
| 895 | gi\|47132623 | NM_145798.2 | oxysterol-binding protein-related protein 7 [Homo sapiens] |
| 896 | gi\|86198309 | NM_001039355.1 | mitochondrial carnitine/acylcarnitine carrier protein CACL [Homo sapiens] |
| 897 | gi\|194097322 | NM_004092.3 | enoyl-CoA hydratase, mitochondrial precursor [Homo sapiens] |
| 898 | gi\|10434001 | AK022548.1 | unnamed protein product [Homo sapiens] |
| 899 | gi\|54792063 | NM_003352.4 | small ubiquitin-related modifier 1 isoform a precursor [Homo sapiens] |
| 900 | gi\|83281439 | NM_003757.2 | eukaryotic translation initiation factor 3 subunit I [Homo sapiens] |
| 901 | gi\|33469915 | NM_003906.3 | 80 kDa MCM3-associated protein [Homo sapiens] |
| 902 | gi\|195972858 | NM_004228.5 | cytohesin-2 isoform 2 [Homo sapiens] |
| 903 | gi\|42716281 | NM_013242.2 | transcription factor IIB [Homo sapiens] |
| 904 | gi\|56676378 | NM_016004.2 | intraflagellar transport protein 52 homolog [Homo sapiens] |
| 905 | gi\|261490711 | NM_138399.4 | transmembrane protein 44 isoform a [Homo sapiens] |
| 906 | gi\|41327758 | NM_001697.2 | ATP synthase subunit O, mitochondrial precursor [Homo sapiens] |
| 907 | gi\|124053441 | NM_000934.3 | alpha-2-antiplasmin isoform a precursor [Homo sapiens] |
| 908 | gi\|47132573 | NM_002733.3 | 5'-AMP-activated protein kinase subunit gamma-1 isoform 1 [Homo sapiens] |
| 909 | gi\|47132588 | NM_002741.3 | serine/threonine-protein kinase N1 isoform 2 [Homo sapiens] |
| 910 | gi\|257196241 | NM_001063.3 | serotransferrin precursor [Homo sapiens] |
| 911 | gi\|205277461 | NM_001064.2 | transketolase [Homo sapiens] |
| 912 | gi\|253795505 | NM_004699.2 | XAP-5 protein [Homo sapiens] |
| 913 | gi\|115527096 | NM_006035.3 | serine/threonine-protein kinase MRCK beta [Homo sapiens] |
| 914 | gi\|68161505 | NM_005718.3 | actin-related protein 2/3 complex subunit 4 isoform a [Homo sapiens] |
| 915 | gi\|194097343 | NM_001114107.2 | PDZ and LIM domain protein 3 isoform b [Homo sapiens] |
| 916 | gi\|32307179 | NM_016139.2 | coiled-coil-helix-coiled-coil-helix domain-containing protein 2, mitochondrial precursor [Homo sapiens] |
| 917 | gi\|170932470 | NM_024650.3 | putative uncharacterized protein C11orf80 [Homo sapiens] |
| 918 | gi\|221218971 | NM_031209.2 | queuine tRNA-ribosyltransferase [Homo sapiens] |
| 919 | gi\|54607109 | NM_174929.2 | zinc finger MIZ domain-containing protein 2 isoform 2 [Homo sapiens] |
| 920 | gi\|58761495 | NM_001011724.1 | heterogeneous nuclear ribonucleoprotein A1-like 2 [Homo sapiens] |
| 921 | gi\|117956372 | NM_001077685.1 | arf-GAP with GTPase, ANK repeat and PH domain-containing protein 7 [Homo sapiens] |
| 922 | gi\|50301237 | NM_000637.2 | glutathione reductase, mitochondrial isoform 1 precursor [Homo sapiens] |
| 923 | gi\|48255890 | NM_001001329.1 | glucosidase 2 subunit beta isoform 2 [Homo sapiens] |
| 924 | gi\|42544245 | NM_003009.2 | selenoprotein W [Homo sapiens] |
| 925 | gi\|6102857 | AL122064.1 | hypothetical protein [Homo sapiens] |
| 926 | gi\|186928845 | NM_032476.3 | 28S ribosomal protein S6, mitochondrial [Homo sapiens] |
| 927 | gi\|19913436 | NM_001694.2 | V-type proton ATPase 16 kDa proteolipid subunit [Homo sapiens] |
| 928 | gi\|205277389 | NM_004082.3 | dynactin subunit 1 isoform 1 [Homo sapiens] |
| 929 | gi\|55750040 | NM_001940.3 | atrophin-1 [Homo sapiens] |
| 930 | gi\|156071491 | NM_002086.4 | growth factor receptor-bound protein 2 isoform 1 [Homo sapiens] |
| 931 | gi\|194018519 | NM_002094.3 | eukaryotic peptide chain release factor GTP-binding subunit ERF3A isoform 1 [Homo sapiens] |
| 932 | gi\|41399283 | NM_002156.4 | 60 kDa heat shock protein, mitochondrial [Homo sapiens] |
| 933 | gi\|116829963 | NM_002256.3 | metastasis-suppressor KiSS-1 [Homo sapiens] |
| 934 | gi\|153945727 | NM_005909.3 | microtubule-associated protein 1B [Homo sapiens] |
| 935 | gi\|189409157 | NM_016841.3 | microtubule-associated protein tau isoform 4 [Homo sapiens] |
| 936 | gi\|14043021 | NM_004990.2 | methionyl-tRNA synthetase, cytoplasmic [Homo sapiens] |
| 937 | gi\|32307122 | NM_004576.2 | serine/threonine-protein phosphatase 2A 55 kDa regulatory subunit B beta isoform isoform a [Homo sapiens] |
| 938 | gi\|22538466 | NM_002796.2 | proteasome subunit beta type-4 [Homo sapiens] |
| 939 | gi\|48762925 | NM_005049.2 | periodic tryptophan protein 2 homolog [Homo sapiens] |
| 940 | gi\|71164881 | NM_001013.3 | 40S ribosomal protein S9 [Homo sapiens] |
| 941 | gi\|171846267 | NM_005726.4 | elongation factor Ts, mitochondrial isoform 2 precursor [Homo sapiens] |
| 942 | gi\|23238209 | NM_005731.2 | actin-related protein 2/3 complex subunit 2 [Homo sapiens] |
| 943 | gi\|14971416 | NM_005762.2 | transcription intermediary factor 1-beta [Homo sapiens] |
| 944 | gi\|207028746 | NM_006096.3 | protein NDRG1 [Homo sapiens] |
| 945 | gi\|22907051 | NM_006409.2 | actin-related protein 2/3 complex subunit 1A isoform 1 [Homo sapiens] |
| 946 | gi\|11055016 | AF142569.1 | hypothetical protein SBBI23 [Homo sapiens] |
| 947 | gi\|112421107 | NM_015125.3 | protein capicua homolog [Homo sapiens] |
| 948 | gi\|56676386 | NM_007364.2 | transmembrane emp24 domain-containing protein 3 precursor [Homo sapiens] |
| 949 | gi\|56676313 | NM_002568.3 | polyadenylate-binding protein 1 [Homo sapiens] |
| 950 | gi\|24431995 | NM_020145.2 | endophilin-B2 [Homo sapiens] |
| 951 | gi\|141802780 | NM_052868.2 | immunoglobulin superfamily member 8 [Homo sapiens] |
| 952 | gi\|17149812 | NM_057161.2 | kelch domain-containing protein 3 isoform 1 [Homo sapiens] |
| 953 | gi\|28274700 | NM_145806.2 | zinc finger protein 511 [Homo sapiens] |
| 954 | gi\|4505488 | NM_002539.1 | ornithine decarboxylase [Homo sapiens] |
| 955 | gi\|78217389 | NM_001004.3 | 60S acidic ribosomal protein P2 [Homo sapiens] |
| 956 | gi\|62739176 | NM_002950.3 | dolichyl-diphosphooligosaccharide--protein glycosyltransferase subunit 1 precursor [Homo sapiens] |
| 957 | gi\|194595508 | NM_001130438.1 | spectrin alpha chain, brain isoform 1 [Homo sapiens] |
| 958 | gi\|13236578 | NM_024330.1 | long-chain fatty acid transport protein 3 [Homo sapiens] |
| 959 | gi\|215277016 | NM_001142370.1 | tyrosine-protein phosphatase non-receptor type 18 isoform 2 [Homo sapiens] |
| 960 | gi\|194018569 | NM_017918.4 | coiled-coil domain-containing protein 109B [Homo sapiens] |
| 961 | gi\|256818771 | NM_018285.3 | U3 small nucleolar ribonucleoprotein protein IMP3 [Homo sapiens] |
| 962 | gi\|209976985 | NM_018386.2 | PCI domain-containing protein 2 [Homo sapiens] |
| 963 | gi\|113722119 | NM_032119.3 | G-protein coupled receptor 98 precursor [Homo sapiens] |
| 964 | gi\|10438604 | AK025936.1 | unnamed protein product [Homo sapiens] |
| 965 | gi\|50234894 | NM_173473.2 | anaphase-promoting complex subunit 16 isoform 1 [Homo sapiens] |
| 966 | gi\|207028465 | NM_005566.3 | L-lactate dehydrogenase A chain isoform 1 [Homo sapiens] |
| 967 | gi\|194440683 | NM_002337.2 | alpha-2-macroglobulin receptor-associated protein precursor [Homo sapiens] |
| 968 | gi\|33356548 | NM_002388.3 | DNA replication licensing factor MCM3 [Homo sapiens] |
| 969 | gi\|28193181 | BX248001.1 | unnamed protein product [Homo sapiens] |
| 970 | gi\|33598947 | NM_002660.2 | 1-phosphatidylinositol-4,5-bisphosphate phosphodiesterase gamma-1 isoform a [Homo sapiens] |
| 971 | gi\|223468675 | NM_021975.3 | transcription factor p65 isoform 1 [Homo sapiens] |
| 972 | gi\|49640008 | NM_003316.3 | E3 ubiquitin-protein ligase TTC3 [Homo sapiens] |
| 973 | gi\|166064032 | NM_145735.2 | rho guanine nucleotide exchange factor 7 isoform b [Homo sapiens] |
| 974 | gi\|83716023 | NM_017596.2 | kinesin-like protein KIF21B [Homo sapiens] |
| 975 | gi\|109148507 | NM_017670.2 | ubiquitin thioesterase OTUB1 [Homo sapiens] |
| 976 | gi\|11034854 | NM_020644.1 | transmembrane protein 9B precursor [Homo sapiens] |
| 977 | gi\|50345990 | NM_001001975.1 | ATP synthase subunit delta, mitochondrial precursor [Homo sapiens] |
| 978 | gi\|145580576 | NM_001329.2 | C-terminal-binding protein 2 isoform 1 [Homo sapiens] |
| 979 | gi\|226958667 | NM_004107.4 | IgG receptor FcRn large subunit p51 precursor [Homo sapiens] |
| 980 | gi\|51476153 | CR749210.1 | hypothetical protein [Homo sapiens] |
| 981 | gi\|193082959 | NM_014487.4 | zinc finger protein 330 [Homo sapiens] |
| 982 | gi\|156416004 | NM_014186.3 | COMM domain-containing protein 9 isoform 1 [Homo sapiens] |

**Tabelle B: Brustkrebs-spezifische Markersequenzen (Aminosäure, Proteine, Peptid) SEQ ID No. 983 - 1473**

| SEQ ID No. | Accession No. | Alias Accesssion No. | Blast |
|---|---|---|---|
| 983 | gi\|261337183 | NP_878908.4 | WAS protein family homolog 1 [Homo sapiens] |
| 984 | gi\|16877438 | AAH16965.1 | NLRP1 protein [Homo sapiens] |
| 985 | gi\|57242761 | NP_003795.2 | receptor-interacting serine/threonine-protein kinase 1 [Homo sapiens] |
| 986 | gi\|54112121 | NP_036558.3 | splicing factor 3B subunit 3 [Homo sapiens] |
| 987 | gi\|21361458 | NP_055601.2 | rho guanine nucleotide exchange factor 17 [Homo sapiens] |
| 988 | gi\|60218897 | NP_005739.2 | YY1-associated factor 2 isoform 2 [Homo sapiens] |
| 989 | gi\|157388906 | NP_060276.2 | Ick-interacting transmembrane adapter 1 [Homo sapiens] |
| 990 | gi\|13569956 | NP_112240.1 | actin-related protein 2/3 complex subunit 5-like protein [Homo sapiens] |
| 991 | gi\|17933772 | NP_525127.1 | protein S100-A16 [Homo sapiens] |
| 992 | gi\|13377002 | NP_008822.2 | gamma-crystallin D [Homo sapiens] |
| 993 | gi\|13325064 | NP_001399.1 | cadherin EGF LAG seven-pass G-type receptor 2 precursor [Homo sapiens] |
| 994 | gi\|55956910 | NP_002421.3 | probable tumor suppressor protein MN1 [Homo sapiens] |
| 995 | gi\|4504245 | NP_003503.1 | histone H2A type 1-C [Homo sapiens] |
| 996 | gi\|77812678 | NP_008834.3 | inorganic pyrophosphatase 2, mitochondrial isoform 2 precursor [Homo sapiens] |
| 997 | gi\|33695078 | NP_037371.2 | serine/threonine-protein phosphatase 2A regulatory subunit B" subunit beta [Homo sapiens] |
| 998 | gi\|56118234 | NP_859067.2 | centromere protein V [Homo sapiens] |
| 999 | gi\|66346713 | NP_001018024.1 | mature T-cell proliferation 1 neighbor protein [Homo sapiens] |
| 1000 | gi\|41352061 | NP_055704.2 | presequence protease, mitochondrial isoform 2 precursor [Homo sapiens] |
| 1001 | gi\|66932990 | NP_001018088.1 | folylpolyglutamate synthase, mitochondrial isoform b [Homo sapiens] |
| 1002 | gi\|31317297 | NP_851998.1 | DNA-binding protein inhibitor ID-1 isoform b [Homo sapiens] |
| 1003 | gi\|29568105 | NP_003683.2 | tomoregulin-1 precursor [Homo sapiens] |
| 1004 | gi\|37181354 | AAQ88491.1 | SEMA5B [Homo sapiens] |
| 1005 | gi\|10864029 | NP_067042.1 | junctional adhesion molecule B precursor [Homo sapiens] |
| 1006 | gi\|42740903 | NP_872307.2 | mTERF domain-containing protein 2 [Homo sapiens] |
| 1007 | gi\|4502743 | NP_001790.1 | cyclin-dependent kinase 7 [Homo sapiens] |
| 1008 | gi\|180637 | AAA90934.1 | L-isoaspartyl/D-aspartyl protein carboxyl methyltransferase [Homo sapiens] |
| 1009 | gi\|67782362 | NP_061903.2 | ATP-dependent RNA helicase DHX29 [Homo sapiens] |
| 1010 | gi\|11036656 | NP_061763.1 | protocadherin beta-7 precursor [Homo sapiens] |
| 1011 | gi\|93204871 | NP_067052.2 | netrin-4 precursor [Homo sapiens] |
| 1012 | gi\|4502495 | NP_001725.1 | complement C1s subcomponent precursor [Homo sapiens] |
| 1013 | gi\|16507950 | NP_055281.2 | tektin-2 [Homo sapiens] |
| 1014 | gi\|75750476 | NP_001028738.1 | mitochondrial Rho GTPase 1 isoform 2 [Homo sapiens] |
| 1015 | gi\|216548627 | NP_001135939.1 | transcription factor HES-4 isoform 1 [Homo sapiens] |
| 1016 | gi\|38683799 | NP_149112.1 | ankyrin repeat domain-containing protein 13A [Homo sapiens] |
| 1017 | gi\|285002231 | NP_001076581.2 | glycerol-3-phosphate dehydrogenase, mitochondrial precursor [Homo sapiens] |
| 1018 | gi\|5454058 | NP_006269.1 | CMP-N-acetylneuraminate-beta-galactosamide-alpha-2,3-sialyltransferase 4 [Homo sapiens] |
| 1019 | gi\|7661964 | NP_055552.1 | ras association domain-containing protein 2 [Homo sapiens] |
| 1020 | gi\|126091095 | NP_001075032.1 | myotonin-protein kinase isoform 1 [Homo sapiens] |
| 1021 | gi\|194097398 | NP_002832.3 | receptor-type tyrosine-protein phosphatase gamma precursor [Homo sapiens] |
| 1022 | gi\|4506775 | NP_003782.1 | membrane-bound transcription factor site-1 protease preproprotein [Homo sapiens] |
| 1023 | gi\|84508631 | NP_077733.3 | CDP-diacylglycerol--glycerol-3-phosphate 3-phosphatidyltransferase, mitochondrial precursor [Homo sapiens] |
| 1024 | gi\|187960073 | NP_006332.3 | mitotic spindle assembly checkpoint protein MAD2B [Homo sapiens] |
| 1025 | gi\|7705706 | NP_057004.1 | 40S ribosomal protein S27-like [Homo sapiens] |
| 1026 | gi\|169234807 | NP_056216.2 | histone deacetylase 7 isoform a [Homo sapiens] |
| 1027 | gi\|120952829 | NP_001073375.1 | zinc finger protein 331 [Homo sapiens] |
| 1028 | gi\|57222570 | NP_612486.2 | codanin-1 [Homo sapiens] |
| 1029 | gi\|21361097 | NP_004116.2 | DNAJC25-GNG10 protein [Homo sapiens] |
| 1030 | gi\|4758796 | NP_004138.1 | developmentally-regulated GTP-binding protein 1 [Homo sapiens] |
| 1031 | gi\|21070997 | NP_003147.2 | stromal interaction molecule 1 precursor [Homo sapiens] |
| 1032 | gi\|42560246 | NP_976035.1 | DNA-directed RNA polymerases I und III subunit RPAC1 [Homo sapiens] |
| 1033 | gi\|114591924 | NP_116780.1 | protein transport protein Sec23B isoform 1 [Homo sapiens] |
| 1034 | gi\|14758316 | NP_004445.1 | ETS translocation variant 5 [Homo sapiens] |
| 1035 | gi\|14504699 | NP_002183.1 | inhibin beta A chain precursor [Homo sapiens] |
| 1036 | gi\|4505293 | NP_002457.1 | myb-related protein B [Homo sapiens] |
| 1037 | gi\|85680426 | ABC72410.1 | BS69 variant 3 [Homo sapiens] |
| 1038 | gi\|19923621 | NP_079469.2 | 3 beta-hydroxysteroid dehydrogenase type 7 isoform a [Homo sapiens] |
| 1039 | gi\|4557515 | NP_000098.1 | DNA damage-binding protein 2 [Homo sapiens] |
| 1040 | gi\|24119203 | NP_705935.1 | tropomyosin alpha-3 chain isoform 2 [Homo sapiens] |
| 1041 | gi\|215820619 | NP_055514.3 | zinc finger protein 646 [Homo sapiens] |
| 1042 | gi\|157502197 | NP_055553.3 | hypothetical protein LOC9772 [Homo sapiens] |
| 1043 | gi\|58331163 | NP_001009936.1 | PHD finger protein 19 isoform b [Homo sapiens] |
| 1044 | gi\|110611233 | NP_569712.2 | collagen alpha-1(XVIII) chain isoform 2 precursor [Homo sapiens] |
| 1045 | gi\|4503151 | NP_000387.1 | cathepsin K preproprotein [Homo sapiens] |
| 1046 | gi\|150417971 | NP_003165.2 | supervillin isoform 1 [Homo sapiens] |
| 1047 | gi\|84872173 | NP_003423.1 | zinc finger protein 131 [Homo sapiens] |
| 1048 | gi\|54792740 | NP_055673.2 | transmembrane and coiled-coil domains protein 2 isoform 1 [Homo sapiens] |
| 1049 | gi\|5031681 | NP_005855.1 | embryonal Fyn-associated substrate isoform 1 [Homo sapiens] |
| 1050 | gi\|148529011 | NP_006082.3 | coronin-2B isoform 1 [Homo sapiens] |
| 1051 | gi\|51317349 | NP_001003789.1 | rab-like protein 2B isoform 1 [Homo sapiens] |
| 1052 | gi\|119120894 | NP_056078.2 | dmX-like protein 2 isoform 2 [Homo sapiens] |
| 1053 | gi\|213972619 | NP_001135445.1 | probable cation-transporting ATPase 13A2 isoform 2 [Homo sapiens] |
| 1054 | gi\|283135240 | NP_064540.3 | integral membrane protein GPR137 isoform 3 [Homo sapiens] |
| 1055 | gi\|13443018 | NP_078847.1 | U11/U12 small nuclear ribonucleoprotein 25 kDa protein [Homo sapiens] |
| 1056 | gi\|45331215 | NP_115805.1 | leucine zipper putative tumor suppressor 2 [Homo sapiens] |
| 1057 | gi\|94721261 | NP_149124.3 | 2',3'-cyclic-nucleotide 3'-phosphodiesterase [Homo sapiens] |
| 1058 | gi\|4504195 | NP_002086.1 | transcription initiation factor IIE subunit beta [Homo sapiens] |
| 1059 | gi\|156616275 | NP_002682.2 | DNA polymerase delta catalytic subunit [Homo sapiens] |
| 1060 | gi\|38146094 | NP_005472.2 | mediator of RNA polymerase II transcription subunit 16 [Homo sapiens] |
| 1061 | gi\|45597177 | NP_055858.2 | TBC1 domain family member 9B isoform b [Homo sapiens] |
| 1062 | gi\|4758050 | NP_004368.1 | carnitine O-palmitoyltransferase 1, muscle isoform isoform a [Homo sapiens] |
| 1063 | gi\|4503063 | NP_000487.1 | beta-crystallin B2 [Homo sapiens] |
| 1064 | gi\|4505785 | NP_000285.1 | phosphorylase b kinase gamma catalytic chain, testis/liver isoform isoform 1 [Homo sapiens] |
| 1065 | gi\|11056044 | NP_066952.1 | inorganic pyrophosphatase [Homo sapiens] |
| 1066 | gi\|18027316 | AAL55740.1 | unknown [Homo sapiens] |
| 1067 | gi\|55770886 | NP_006528.2 | ubiquitin carboxyl-terminal hydrolase 3 [Homo sapiens] |
| 1068 | gi\|31742478 | NP_006306.2 | polycomb group RING finger protein 3 [Homo sapiens] |
| 1069 | gi\|2224667 | BAA20818.1 | KIAA0363 [Homo sapiens] |
| 1070 | gi\|33356550 | NP_872620.1 | G patch domain-containing protein 4 isoform 2 [Homo sapiens] |
| 1071 | gi\|11641289 | NP_071761.1 | dimethyladenosine transferase 2, mitochondrial [Homo sapiens] |
| 1072 | gi\|48762920 | NP_002617.3 | 6-phosphofructokinase, liver type [Homo sapiens] |
| 1073 | gi\|5453908 | NP_006215.1 | phosphatidylinositol transfer protein alpha isoform [Homo sapiens] |
| 1074 | gi\|5032173 | NP_005645.1 | COUP transcription factor 1 [Homo sapiens] |
| 1075 | gi\|4758984 | NP_004654.1 | ras-related protein Rab-11A isoform 1 [Homo sapiens] |
| 1076 | gi\|150378439 | NP_064611.3 | PR domain zinc finger protein 8 [Homo sapiens] |
| 1077 | gi\|33147080 | NP_065850.1 | AMSH-like protease [Homo sapiens] |
| 1078 | gi\|4504025 | NP_002055.1 | glutaredoxin-1 [Homo sapiens] |
| 1079 | gi\|4504517 | NP_001531.1 | heat shock protein beta-1 [Homo sapiens] |
| 1080 | gi\|20149568 | NP_009034.2 | NADH dehydrogenase [ubiquinone] flavoprotein 1, mitochondrial isoform 1 precursor [Homo sapiens] |
| 1081 | gi\|48255924 | NP_066553.3 | NADH dehydrogenase [ubiquinone] flavoprotein 3, mitochondrial isoform a precursor [Homo sapiens] |
| 1082 | gi\|8923892 | NP_061133.1 | peroxisomal membrane protein 2 [Homo sapiens] |
| 1083 | gi\|117553584 | NP_001070991.1 | AP-3 complex subunit delta-1 isoform 1 [Homo sapiens] |
| 1084 | gi\|7661952 | NP_055521.1 | squamous cell carcinoma antigen recognized by T-cells 3 [Homo sapiens] |
| 1085 | gi\|5453718 | NP_006402.1 | 1-acyl-sn-glycerol-3-phosphate acyltransferase alpha [Homo sapiens] |
| 1086 | gi\|5453734 | NP_006445.1 | max dimerization protein 4 [Homo sapiens] |
| 1087 | gi\|187960090 | NP_056142.2 | protein SMG5 [Homo sapiens] |
| 1088 | gi\|7662018 | NP_055968.1 | PHD finger protein 3 [Homo sapiens] |
| 1089 | gi\|187761373 | NP_005735.2 | E3 ubiquitin-protein ligase ARIH1 [Homo sapiens] |
| 1090 | gi\|102467484 | NP_060221.2 | sphingomyelin phosphodiesterase 4 isoform 1 [Homo sapiens] |
| 1091 | gi\|9910354 | NP_064532.1 | THAP domain-containing protein 10 [Homo sapiens] |
| 1092 | gi\|214010240 | NP_001135764.1 | VIP36-like protein isoform 1 [Homo sapiens] |
| 1093 | gi\|14249672 | NP_116291.1 | protein phosphatase 1 regulatory subunit 16A [Homo sapiens] |
| 1094 | gi\|333609251 | NP_001005850.2 | zinc finger protein 835 [Homo sapiens] |
| 1095 | gi\|53759122 | NP_000029.2 | adenomatous polyposis coli protein isoform b [Homo sapiens] |
| 1096 | gi\|87578392 | NP_114033.2 | microtubule-associated protein 2 isoform 2 [Homo sapiens] |
| 1097 | gi\|5729862 | NP_006518.1 | histone RNA hairpin-binding protein [Homo sapiens] |
| 1098 | gi\|5803181 | NP_006810.1 | stress-induced-phosphoprotein 1 [Homo sapiens] |
| 1099 | gi\|17978485 | NP_065908.1 | vacuolar protein sorting-associated protein 18 homolog [Homo sapiens] |
| 1100 | gi\|166795299 | NP_006507.2 | solute carrier family 2, facilitated glucose transporter member 1 [Homo sapiens] |
| 1101 | gi\|7661886 | NP_055579.1 | DAZ-associated protein 2 isoform a [Homo sapiens] |
| 1102 | gi\|148612829 | NP_001091979.1 | small G protein signaling modulator 2 isoform 2 [Homo sapiens] |
| 1103 | gi\|136796743 | NP_056255.2 | monofunctional C1-tetrahydrofolate synthase, mitochondrial isoform 2 precursor [Homo sapiens] |
| 1104 | gi\|21361734 | NP_060461.3 | hypothetical protein LOC55683 isoform b [Homo sapiens] |
| 1105 | gi\|70778869 | NP_068752.2 | migration and invasion-inhibitory protein [Homo sapiens] |
| 1106 | gi\|38176285 | NP_115569.2 | ribonuclease H2 subunit C [Homo sapiens] |
| 1107 | gi\|24475814 | NP_612211.1 | DNA-directed RNA polymerase III subunit RPC8 isoform a [Homo sapiens] |
| 1108 | gi\|21699088 | NP_660338.1 | zinc finger protein 627 [Homo sapiens] |
| 1109 | gi\|21264343 | NP_002958.2 | scaffold attachment factor B1 isoform 3 [Homo sapiens] |
| 1110 | gi\|5454064 | NP_006319.1 | RNA-binding protein 14 isoform 1 [Homo sapiens] |
| 1111 | gi\|149588534 | NP_001092303.1 | ataxin-7-like protein 3 isoform b [Homo sapiens] |
| 1112 | gi\|20270357 | NP_620157.1 | AN1-type zinc finger protein 2B [Homo sapiens] |
| 1113 | gi\|35493701 | NP_689777.3 | vacuolar protein sorting-associated protein 13B isoform 1 [Homo sapiens] |
| 1114 | gi\|118130809 | NP_001071161.1 | endothelial cell-specific chemotaxis regulator precursor [Homo sapiens] |
| 1115 | gi\|71773208 | NP_000674.2 | alpha-2C adrenergic receptor [Homo sapiens] |
| 1116 | gi\|55925576 | NP_000588.2 | insulin-like growth factor-binding protein 2 precursor [Homo sapiens] |
| 1117 | gi\|71772583 | NP_001025172.1 | 40S ribosomal protein S29 isoform 2 [Homo sapiens] |
| 1118 | gi\|18105054 | NP_055785.2 | kinesin-associated protein 3 isoform 1 [Homo sapiens] |
| 1119 | gi\|13489112 | NP_055153.1 | phosphatidylserine decarboxylase proenzyme [Homo sapiens] |
| 1120 | gi\|12060857 | AAG48270.1 | serologically defined breast cancer antigen NY-BR-99 [Homo sapiens] |
| 1121 | gi\|7020625 | BAA91205.1 | unnamed protein product [Homo sapiens] |
| 1122 | gi\|32698884 | NP_872333.1 | calcium-binding protein 7 [Homo sapiens] |
| 1123 | gi\|56090146 | NP_001005920.2 | jmjC domain-containing protein 8 [Homo sapiens] |
| 1124 | gi\|166235895 | NP_001107561.1 | ectonucleoside triphosphate diphosphohydrolase 6 isoform 2 [Homo sapiens] |
| 1125 | gi\|189571687 | NP_003722.2 | Sjoegren syndrome nuclear autoantigen 1 [Homo sapiens] |
| 1126 | gi\|22035581 | NP_665801.1 | septin-6 isoform D [Homo sapiens] |
| 1127 | gi\|98961133 | NP_079388.3 | zinc finger protein ZXDC isoform 1 [Homo sapiens] |
| 1128 | gi\|66267730 | NP_443076.2 | WD repeat-containing protein 34 [Homo sapiens] |
| 1129 | gi\|19923971 | NP_612452.1 | HAUS augmin-like complex subunit 1 [Homo sapiens] |
| 1130 | gi\|5453579 | NP_006120.1 | bone morphogenetic protein 1 isoform 3 precursor [Homo sapiens] |
| 1131 | gi\|161727458 | BAF94339.1 | dihydropyrimidinase-like 2 long form [Homo sapiens] |
| 1132 | gi\|14702171 | NP_116558.1 | DNA-directed RNA polymerase II subunit RPB3 [Homo sapiens] |
| 1133 | gi\|4507691 | NP_003487.1 | transformation/transcription domain-associated protein [Homo sapiens] |
| 1134 | gi\|10863889 | NP_005137.1 | U4/U6.U5 tri-snRNP-associated protein 1 [Homo sapiens] |
| 1135 | gi\|38201692 | NP_031394.2 | ras GTPase-activating protein 3 [Homo sapiens] |
| 1136 | gi\|7657455 | NP_055118.1 | pescadillo homolog [Homo sapiens] |
| 1137 | gi\|28416431 | NP_056475.1 | GTPase IMAP family member 2 [Homo sapiens] |
| 1138 | gi\|118498364 | NP_001072988.1 | dapper homolog 1 isoform 2 [Homo sapiens] |
| 1139 | gi\|14150203 | NP_115753.1 | transcription elongation factor 1 homolog [Homo sapiens] |
| 1140 | gi\|11321585 | NP_002065.1 | guanine nucleotide-binding protein G(I)/G(S)/G(T) subunit beta-1 [Homo sapiens] |
| 1141 | gi\|4504183 | NP_000843.1 | glutathione S-transferase P [Homo sapiens] |
| 1142 | gi\|46255047 | NP_001526.2 | protein arginine N-methyltransferase 2 isoform 1 [Homo sapiens] |
| 1143 | gi\|157412270 | NP_112480.2 | heterogeneous nuclear ribonucleoprotein M isoform b [Homo sapiens] |
| 1144 | gi\|5453766 | NP_006150.1 | protein kinase C-binding protein NELL2 isoform b precursor [Homo sapiens] |
| 1145 | gi\|57863259 | NP_001008897.1 | T-complex protein 1 subunit alpha isoform b [Homo sapiens] |
| 1146 | gi\|9994175 | NP_003769.1 | beta-1,4-galactosyltransferase 4 [Homo sapiens] |
| 1147 | gi\|145611426 | NP_006805.2 | proteasome inhibitor PI31 subunit [Homo sapiens] |
| 1148 | gi\|17999539 | NP_054722.2 | pre-mRNA-splicing factor ATP-dependent RNA helicase PRP16 [Homo sapiens] |
| 1149 | gi\|5453601 | NP_006362.1 | cartilage-associated protein precursor [Homo sapiens] |
| 1150 | gi\|40807485 | NP_036601.2 | pre-mRNA-processing factor 6 [Homo sapiens] |
| 1151 | gi\|24308440 | NP_612410.1 | multivesicular body subunit 12A [Homo sapiens] |
| 1152 | gi\|28827795 | NP_789782.1 | charged multivesicular body protein 4b [Homo sapiens] |
| 1153 | gi\|32964825 | NP_689945.2 | radial spoke head protein 9 homolog isoform 1 [Homo sapiens] |
| 1154 | gi\|320089576 | NP_001004333.2 | ribonuclease kappa [Homo sapiens] |
| 1155 | gi\|4557235 | NP_000009.1 | very long-chain specific acyl-CoA dehydrogenase, mitochondrial isoform 1 precursor [Homo sapiens] |
| 1156 | gi\|21618334 | NP_003994.2 | carnitine acetyltransferase isoform 2 [Homo sapiens] |
| 1157 | gi\|38201714 | NP_001410.2 | ELAV-like protein 1 [Homo sapiens] |
| 1158 | gi\|296531408 | NP_937798.3 | ES1 protein homolog, mitochondrial isoform Ib precursor [Homo sapiens] |
| 1159 | gi\|22027642 | NP_036421.2 | kelch-like ECH-associated protein 1 [Homo sapiens] |
| 1160 | gi\|141872646 | NP_055595.2 | cullin-7 isoform 2 [Homo sapiens] |
| 1161 | gi\|42544179 | NP_036259.2 | cip1-interacting zinc finger protein isoform 1 [Homo sapiens] |
| 1162 | gi\|7705716 | NP_057037.1 | nitric oxide synthase-interacting protein [Homo sapiens] |
| 1163 | gi\|124256478 | NP_060444.3 | autophagy-related protein 16-1 isoform 2 [Homo sapiens] |
| 1164 | gi\|110349769 | NP_077272.2 | coiled-coil domain-containing protein 28B [Homo sapiens] |
| 1165 | gi\|40555765 | AAH64481.1 | RNF187 protein [Homo sapiens] |
| 1166 | gi\|194385888 | BAG65319.1 | unnamed protein product [Homo sapiens] |
| 1167 | gi\|113930754 | NP_001039021.1 | src-like-adapter isoform a [Homo sapiens] |
| 1168 | gi\|19557695 | NP_598395.1 | mediator of RNA polymerase II transcription subunit 22 isoform b [Homo sapiens] |
| 1169 | gi\|14507725 | NP_000362.1 | transthyretin precursor [Homo sapiens] |
| 1170 | gi\|36287060 | NP_874368.1 | histone acetyltransferase KAT5 isoform 3 [Homo sapiens] |
| 1171 | gi\|58331185 | NP_001009570.1 | T-complex protein 1 subunit eta isoform b [Homo sapiens] |
| 1172 | gi\|42544142 | NP_974223.1 | PDZ domain-containing protein GIPC1 isoform 2 [Homo sapiens] |
| 1173 | gi\|88501740 | NP_008963.3 | TRIO and F-actin-binding protein isoform 1 [Homo sapiens] |
| 1174 | gi\|66472382 | NP_009115.2 | nischarin [Homo sapiens] |
| 1175 | gi\|20143912 | NP_599027.1 | WD repeat and SOCS box-containing protein 1 isoform 2 [Homo sapiens] |
| 1176 | gi\|23308577 | NP_006614.2 | D-3-phosphoglycerate dehydrogenase [Homo sapiens] |
| 1177 | gi\|7657381 | NP_055317.1 | pre-mRNA-processing factor 19 [Homo sapiens] |
| 1178 | gi\|117553615 | NP_057129.2 | protein slowmo homolog 2 [Homo sapiens] |
| 1179 | gi\|7706089 | NP_057642.1 | SCAN domain-containing protein 1 isoform 1 [Homo sapiens] |
| 1180 | gi\|74048434 | NP_057393.2 | leucine carboxyl methyltransferase 1 isoform a [Homo sapiens] |
| 1181 | gi\|8922735 | NP_060725.1 | elongator complex protein 2 isoform 2 [Homo sapiens] |
| 1182 | gi\|145275200 | NP_079356.3 | L-asparaginase [Homo sapiens] |
| 1183 | gi\|42734379 | NP_612359.2 | THAP domain-containing protein 3 isoform 2 [Homo sapiens] |
| 1184 | gi\|4502619 | NP_001751.1 | G1/S-specific cyclin-D3 isoform 2 [Homo sapiens] |
| 1185 | gi\|4826708 | NP_004943.1 | ephrin-A3 precursor [Homo sapiens] |
| 1186 | gi\|4506971 | NP_003040.1 | sodium/bile acid cotransporter [Homo sapiens] |
| 1187 | gi\|284005309 | NP_004251.3 | ATP-dependent DNA helicase Q4 [Homo sapiens] |
| 1188 | gi\|24475816 | NP_612510.1 | trans-2,3-enoyl-CoA reductase [Homo sapiens] |
| 1189 | gi\|45430055 | NP_055494.2 | centrosomal protein of 57 kDa [Homo sapiens] |
| 1190 | gi\|45643119 | NP_006108.2 | enoyl-CoA delta isomerase 2, mitochondrial isoform 1 [Homo sapiens] |
| 1191 | gi\|21361647 | NP_006612.2 | putative adenosylhomocysteinase 2 isoform a [Homo sapiens] |
| 1192 | gi\|33469978 | NP_878255.1 | sec1 family domain-containing protein 1 isoform b [Homo sapiens] |
| 1193 | gi\|118200356 | NP_055209.2 | growth hormone-inducible transmembrane protein [Homo sapiens] |
| 1194 | gi\|59850649 | NP_060913.2 | zinc finger protein 302 [Homo sapiens] |
| 1195 | gi\|31415880 | NP_852606.1 | protein phosphatase 1 regulatory subunit 1B isoform 2 [Homo sapiens] |
| 1196 | gi\|12669911 | NP_005216.1 | transcription factor E2F1 [Homo sapiens] |
| 1197 | gi\|51102291 | NP_005386.2 | postmeiotic segregation increased 2-like 3 isoform 1 [Homo sapiens] |
| 1198 | gi\|148255970 | NP_002960.2 | mitogen-activated protein kinase 12 [Homo sapiens] |
| 1199 | gi\|7657134 | NP_055051.1 | dihydroxyacetone phosphate acyltransferase [Homo sapiens] |
| 1200 | gi\|9558729 | NP_037497.1 | ADP-ribosylation factor-binding protein GGA1 isoform 1 [Homo sapiens] |
| 1201 | gi\|213385323 | NP_001131031.1 | anaphase-promoting complex subunit 5 isoform b [Homo sapiens] |
| 1202 | gi\|7706557 | NP_057604.1 | chromosome 9 open reading frame 78 [Homo sapiens] |
| 1203 | gi\|37537687 | NP_060807.2 | zinc finger protein 444 [Homo sapiens] |
| 1204 | gi\|68533249 | NP_060946.3 | protein BEX1 [Homo sapiens] |
| 1205 | gi\|192449449 | NP_071349.3 | ubiquitin-conjugating enzyme E2 O [Homo sapiens] |
| 1206 | gi\|12232385 | NP_073567.1 | COP9 signalosome complex subunit 7b [Homo sapiens] |
| 1207 | gi\|13236587 | NP_077310.1 | transmembrane protein 43 [Homo sapiens] |
| 1208 | gi\|19923359 | NP_006244.2 | 5'-AMP-activated protein kinase subunit beta-1 [Homo sapiens] |
| 1209 | gi\|29826335 | NP_003899.2 | eukaryotic translation initiation factor 2 subunit 2 [Homo sapiens] |
| 1210 | gi\|237649015 | NP_006815.2 | probable rRNA-processing protein EBP2 isoform 2 [Homo sapiens] |
| 1211 | gi\|6005846 | NP_009215.1 | twinfilin-2 [Homo sapiens] |
| 1212 | gi\|39780571 | NP_060586.2 | protein misato homolog 1 [Homo sapiens] |
| 1213 | gi\|40068485 | NP_073745.2 | von Willebrand factor A domain-containing protein 1 isoform 1 precursor [Homo sapiens] |
| 1214 | gi\|217416379 | NP_001136122.1 | heterogeneous nuclear ribonucleoprotein L-like isoform 2 [Homo sapiens] |
| 1215 | gi\|71834872 | NP_849196.2 | serine incorporator 2 isoform 1 [Homo sapiens] |
| 1216 | gi\|55749577 | NP_001142.2 | ADP/ATP translocase 1 [Homo sapiens] |
| 1217 | gi\|4503599 | NP_001974.1 | DNA excision repair protein ERCC-1 isoform 2 [Homo sapiens] |
| 1218 | gi\|4504279 | NP_002098.1 | histone H3.3 [Homo sapiens] |
| 1219 | gi\|5453555 | NP_006316.1 | GTP-binding nuclear protein Ran [Homo sapiens] |
| 1220 | gi\|23111018 | NP_690595.1 | RNA-binding protein 10 isoform 2 [Homo sapiens] |
| 1221 | gi\|157785645 | NP_005867.3 | striated muscle preferentially expressed protein kinase isoform 1 [Homo sapiens] |
| 1222 | gi\|23397429 | NP_006351.2 | eukaryotic translation initiation factor 3 subunit M [Homo sapiens] |
| 1223 | gi\|197100773 | NP_064716.2 | probable arginyl-tRNA synthetase, mitochondrial precursor [Homo sapiens] |
| 1224 | gi\|89337270 | NP_073586.5 | retinoic acid induced 16 [Homo sapiens] |
| 1225 | gi\|47174859 | NP_079334.3 | tripartite motif-containing protein 46 [Homo sapiens] |
| 1226 | gi\|109638743 | NP_113666.2 | hypothetical protein LOC83723 [Homo sapiens] |
| 1227 | gi\|53729359 | NP_612370.3 | E3 ubiquitin-protein ligase LRSAM1 isoform 1 [Homo sapiens] |
| 1228 | gi\|4506027 | NP_002711.1 | serine/threonine-protein phosphatase 4 catalytic subunit [Homo sapiens] |
| 1229 | gi\|4507509 | NP_003245.1 | metalloproteinase inhibitor 1 precursor [Homo sapiens] |
| 1230 | gi\|109150416 | NP_036425.1 | peroxidasin homolog precursor [Homo sapiens] |
| 1231 | gi\|34365371 | CAE46007.1 | hypothetical protein [Homo sapiens] |
| 1232 | gi\|6005794 | NP_009144.1 | PRA1 family protein 2 [Homo sapiens] |
| 1233 | gi\|33859678 | NP_060349.1 | zinc finger protein 416 [Homo sapiens] |
| 1234 | gi\|149274653 | NP_065831.1 | patched domain-containing protein 2 [Homo sapiens] |
| 1235 | gi\|50511941 | NP_597712.2 | EMI domain-containing protein 1 [Homo sapiens] |
| 1236 | gi\|14502205 | NP_001651.1 | ADP-ribosylation factor 4 [Homo sapiens] |
| 1237 | gi\|34335194 | NP_874389.1 | cAMP-responsive element modulator isoform g [Homo sapiens] |
| 1238 | gi\|4503093 | NP_001885.1 | casein kinase I isoform epsilon [Homo sapiens] |
| 1239 | gi\|20357529 | NP_005264.2 | guanine nucleotide-binding protein G(I)/G(S)/G(T) subunit beta-2 [Homo sapiens] |
| 1240 | gi\|188497750 | NP_277035.2 | hexokinase-1 isoform HKI-td [Homo sapiens] |
| 1241 | gi\|5031905 | NP_005577.1 | myoD family inhibitor [Homo sapiens] |
| 1242 | gi\|38679892 | NP_006214.2 | peptidyl-prolyl cis-trans isomerase NIMA-interacting 4 isoform 1 [Homo sapiens] |
| 1243 | gi\|4502483 | NP_003466.1 | ras association domain-containing protein 7 isoform 1 [Homo sapiens] |
| 1244 | gi\|4507947 | NP_003671.1 | tyrosyl-tRNA synthetase, cytoplasmic [Homo sapiens] |
| 1245 | gi\|4757772 | NP_004666.1 | GTP-binding protein Di-Ras3 [Homo sapiens] |
| 1246 | gi\|150170699 | NP_056471.1 | kinesin-like protein KIF26A [Homo sapiens] |
| 1247 | gi\|7705294 | NP_057272.1 | actin-like protein 6B [Homo sapiens] |
| 1248 | gi\|49574502 | NP_057327.2 | NADH-cytochrome b5 reductase 1 [Homo sapiens] |
| 1249 | gi\|13027612 | NP_076429.1 | ras-like protein family member 11B [Homo sapiens] |
| 1250 | gi\|55749600 | NP_115827.1 | protein spire homolog 2 [Homo sapiens] |
| 1251 | gi\|39992616 | AAH64477.1 | PIM3 protein [Homo sapiens] |
| 1252 | gi\|56118217 | NP_001008217.1 | UDP-glucose 4-epimerase [Homo sapiens] |
| 1253 | gi\|27502383 | NP_758527.1 | homeobox protein Meis2 isoform h [Homo sapiens] |
| 1254 | gi\|91208423 | NP_003293.2 | thyroid receptor-interacting protein 6 [Homo sapiens] |
| 1255 | gi\|154800449 | NP_003424.3 | zinc finger protein 132 [Homo sapiens] |
| 1256 | gi\|32189362 | NP_003651.1 | liprin-alpha-3 [Homo sapiens] |
| 1257 | gi\|5803072 | NP_006760.1 | LIM domain transcription factor LMO4 [Homo sapiens] |
| 1258 | gi\|253735775 | NP_001155855.1 | rho guanine nucleotide exchange factor 2 isoform 1 [Homo sapiens] |
| 1259 | gi\|5730027 | NP_006550.1 | KH domain-containing, RNA-binding, signal transduction-associated protein 1 [Homo sapiens] |
| 1260 | gi\|219555665 | NP_001137252.1 | solute carrier family 25 member 39 isoform a [Homo sapiens] |
| 1261 | gi\|8922297 | NP_060502.1 | putative RNA-binding protein Luc7-like 1 isoform a [Homo sapiens] |
| 1262 | gi\|149274624 | NP_112177.2 | protein LBH [Homo sapiens] |
| 1263 | gi\|58761548 | NP_115927.1 | tau-tubulin kinase 1 [Homo sapiens] |
| 1264 | gi\|15529978 | NP_219482.1 | zinc finger protein 622 [Homo sapiens] |
| 1265 | gi\|304555614 | NP_689956.2 | BRCA1-associated ATM activator 1 [Homo sapiens] |
| 1266 | gi\|32171205 | NP_859074.1 | EF-hand domain-containing family member A2 [Homo sapiens] |
| 1267 | gi\|119964728 | NP_689996.4 | D-2-hydroxyglutarate dehydrogenase, mitochondrial precursor [Homo sapiens] |
| 1268 | gi\|109148542 | NP_001596.2 | alanyl-tRNA synthetase, cytoplasmic [Homo sapiens] |
| 1269 | gi\|83700235 | NP_001958.2 | eukaryotic initiation factor 4A-II [Homo sapiens] |
| 1270 | gi\|20127408 | NP_000173.2 | trifunctional enzyme subunit alpha, mitochondrial precursor [Homo sapiens] |
| 1271 | gi\|6996014 | NP_002100.2 | histidyl-tRNA synthetase, cytoplasmic [Homo sapiens] |
| 1272 | gi\|17105394 | NP_000975.2 | 60S ribosomal protein L23a [Homo sapiens] |
| 1273 | gi\|4506605 | NP_000969.1 | 60S ribosomal protein L23 [Homo sapiens] |
| 1274 | gi\|45446743 | NP_031398.2 | ATP-dependent RNA helicase DDX42 [Homo sapiens] |
| 1275 | gi\|39725634 | NP_056130.2 | la-related protein 1 isoform 1 [Homo sapiens] |
| 1276 | gi\|112382377 | NP_055316.2 | ubiquitin-conjugating enzyme E2 S [Homo sapiens] |
| 1277 | gi\|82830424 | NP_001032622.1 | GON-4-like protein isoform a [Homo sapiens] |
| 1278 | gi\|13129128 | NP_077017.1 | hypothetical protein LOC79095 [Homo sapiens] |
| 1279 | gi\|40548332 | NP_954981.1 | coiled-coil domain-containing protein 137 [Homo sapiens] |
| 1280 | gi\|194353970 | NP_000672.3 | alpha-2A adrenergic receptor [Homo sapiens] |
| 1281 | gi\|4757886 | NP_004330.1 | pituitary tumor-transforming gene 1 protein-interacting protein precursor [Homo sapiens] |
| 1282 | gi\|21237748 | NP_004348.2 | CD151 antigen [Homo sapiens] |
| 1283 | gi\|4502923 | NP_001830.1 | calponin-3 [Homo sapiens] |
| 1284 | gi\|20070199 | NP_005225.2 | nuclear receptor subfamily 2 group F member 6 [Homo sapiens] |
| 1285 | gi\|9845511 | NP_008839.2 | ras-related C3 botulinum toxin substrate 1 isoform Rac1 [Homo sapiens] |
| 1286 | gi\|4507793 | NP_003339.1 | ubiquitin-conjugating enzyme E2 N [Homo sapiens] |
| 1287 | gi\|4507987 | NP_003428.1 | zinc finger protein 136 [Homo sapiens] |
| 1288 | gi\|197333755 | NP_006755.4 | interferon-related developmental regulator 2 [Homo sapiens] |
| 1289 | gi\|23308699 | NP_005452.2 | transcription factor MafB [Homo sapiens] |
| 1290 | gi\|114796626 | NP_037488.2 | monocarboxylate transporter 3 [Homo sapiens] |
| 1291 | gi\|18496983 | NP_056341.1 | CAP-Gly domain-containing linker protein 3 [Homo sapiens] |
| 1292 | gi\|323635446 | NP_065127.4 | zinc finger protein 695 isoform 1 [Homo sapiens] |
| 1293 | gi\|92087055 | NP_072096.2 | PERQ amino acid-rich with GYF domain-containing protein 1 [Homo sapiens] |
| 1294 | gi\|186928847 | NP_116219.2 | cirhin [Homo sapiens] |
| 1295 | gi\|62241011 | NP_005154.2 | RAC-alpha serine/threonine-protein kinase [Homo sapiens] |
| 1296 | gi\|4502337 | NP_001176.1 | zinc-alpha-2-glycoprotein precursor [Homo sapiens] |
| 1297 | gi\|21735621 | NP_005909.2 | malate dehydrogenase, mitochondrial precursor [Homo sapiens] |
| 1298 | gi\|115387094 | NP_002991.2 | succinate dehydrogenase [ubiquinone] iron-sulfur subunit, mitochondrial precursor [Homo sapiens] |
| 1299 | gi\|54607089 | NP_001005914.1 | semaphorin-3B isoform 2 precursor [Homo sapiens] |
| 1300 | gi\|56550051 | NP_006581.2 | U4/U6.U5 tri-snRNP-associated protein 2 [Homo sapiens] |
| 1301 | gi34147637 | NP_006824.2 | COP9 signalosome complex subunit 6 [Homo sapiens] |
| 1302 | gi11056036 | NP_055955.1 | tubulin--tyrosine ligase-like protein 12 [Homo sapiens] |
| 1303 | gi10047104 | NP_057389.1 | SS18-like protein 2 [Homo sapiens] |
| 1304 | gi32129199 | NP_149073.1 | SAP domain-containing ribonucleoprotein [Homo |
| | | | sapiens] |
| 1305 | gi\|40548382 | NP_954857.1 | multidrug resistance-related protein [Homo sapiens] |
| 1306 | gi\|45580696 | NP_996261.1 | hematopoietic signal peptide-containing isoform 2 [Homo sapiens] |
| 1307 | gi\|19913441 | NP_002140.2 | hippocalcin-like protein 1 [Homo sapiens] |
| 1308 | gi\|316983124 | NP_066357.2 | 60S ribosomal protein L36a isoform a [Homo sapiens] |
| 1309 | gi\|29568086 | NP_002988.3 | syndecan-1 precursor [Homo sapiens] |
| 1310 | gi\|4507847 | NP_003358.1 | upstream stimulatory factor 2 isoform 1 [Homo sapiens] |
| 1311 | gi\|88853069 | NP_000629.3 | vitronectin precursor [Homo sapiens] |
| 1312 | gi\|4503825 | NP_003496.1 | frizzled-1 precursor [Homo sapiens] |
| 1313 | gi\|6912274 | NP_036241.1 | transmembrane protein 59-like precursor [Homo sapiens] |
| 1314 | gi\|151101235 | NP_036593.2 | TERF1-interacting nuclear factor 2 isoform 2 [Homo sapiens] |
| 1315 | gi\|9945322 | NP_064705.1 | vesicular glutamate transporter 1 [Homo sapiens] |
| 1316 | gi\|165932370 | NP_078858.4 | protocadherin Fat 4 precursor [Homo sapiens] |
| 1317 | gi\|52353306 | NP_001005210.1 | leucine-rich repeat-containing protein 55 [Homo sapiens] |
| 1318 | gi\|194378374 | BAG57937.1 | unnamed protein product [Homo sapiens] |
| 1319 | gi\|32454741 | NP_001226.2 | serpin H1 precursor [Homo sapiens] |
| 1320 | gi\|5031631 | NP_005497.1 | lysosome membrane protein 2 isoform 1 [Homo sapiens] |
| 1321 | gi\|18201905 | NP_000166.2 | glucose-6-phosphate isomerase isoform 2 [Homo sapiens] |
| 1322 | gi\|14758774 | NP_004539.1 | NADH dehydrogenase [ubiquinone] 1 beta subcomplex subunit 10 [Homo sapiens] |
| 1323 | gi\|4507669 | NP_003286.1 | translationally-controlled tumor protein [Homo sapiens] |
| 1324 | gi\|23397696 | NP_690902.1 | copine-1 isoform a [Homo sapiens] |
| 1325 | gi\|5031569 | NP_005727.1 | alpha-centractin [Homo sapiens] |
| 1326 | gi\|21361358 | NP_006365.2 | serine/threonine-protein kinase 25 [Homo sapiens] |
| 1327 | gi\|5803167 | NP_006793.1 | splicing factor 3A subunit 3 [Homo sapiens] |
| 1328 | gi\|24307991 | NP_055904.1 | cullin-9 [Homo sapiens] |
| 1329 | gi\|41872443 | NP_955400.1 | short transient receptor potential channel 4-associated protein isoform b [Homo sapiens] |
| 1330 | gi\|4557010 | NP_001601.1 | lysosomal acid phosphatase isoform 1 precursor [Homo sapiens] |
| 1331 | gi\|169636439 | NP_000069.2 | cholesteryl ester transfer protein precursor [Homo sapiens] |
| 1332 | gi\|4504193 | NP_001505.1 | transcription initiation factor IIB [Homo sapiens] |
| 1333 | gi\|63253298 | NP_003123.2 | spermidine synthase [Homo sapiens] |
| 1334 | gi\|7705266 | NP_057537.1 | NCK-interacting protein with SH3 domain isoform 1 [Homo sapiens] |
| 1335 | gi\|37622894 | NP_919442.1 | RING finger protein 126 [Homo sapiens] |
| 1336 | gi\|13540531 | NP_110395.1 | integrin-linked kinase-associated serine/threonine phosphatase 2C [Homo sapiens] |
| 1337 | gi\|14150141 | NP_115722.1 | programmed cell death protein 2-like [Homo sapiens] |
| 1338 | gi\|119120877 | NP_597731.2 | zinc finger protein 721 [Homo sapiens] |
| 1339 | gi\|4826659 | NP_004921.1 | F-actin-capping protein subunit beta isoform 1 [Homo sapiens] |
| 1340 | gi\|4758356 | NP_004102.1 | flap endonuclease 1 [Homo sapiens] |
| 1341 | gi\|19923142 | NP_002256.2 | importin subunit beta-1 [Homo sapiens] |
| 1342 | gi\|4557707 | NP_000416.1 | neural cell adhesion molecule L1 isoform 1 precursor [Homo sapiens] |
| 1343 | gi\|4505749 | NP_000280.1 | 6-phosphofructokinase, muscle type isoform 2 [Homo sapiens] |
| 1344 | gi\|4505941 | NP_000929.1 | DNA-directed RNA polymerase II subunit RPB2 [Homo sapiens] |
| 1345 | gi\|156631005 | NP-002803.2 | 26S proteasome non-ATPase regulatory subunit 8 [Homo sapiens] |
| 1346 | gi\|21264353 | NP_003069.2 | SWI/SNF-related matrix-associated actin-dependent regulator of chromatin subfamily D member 3 isoform 1 [Homo sapiens] |
| 1347 | gi\|4507385 | NP_003186.1 | transcription elongation factor A protein 2 isoform a [Homo sapiens] |
| 1348 | gi\|63162572 | NP_005989.3 | T-complex protein 1 subunit gamma isoform a [Homo sapiens] |
| 1349 | gi\|55769587 | NP_003694.1 | nucleolar protein 14 [Homo sapiens] |
| 1350 | gi\|95147538 | NP_006042.3 | amyloid beta A4 precursor protein-binding family B member 3 isoform d [Homo sapiens] |
| 1351 | gi\|8922333 | NP_060519.1 | pleckstrin homology domain-containing family J member 1 [Homo sapiens] |
| 1352 | gi\|8923881 | NP_061109.1 | WD repeat and SOCS box-containing protein 2 [Homo sapiens] |
| 1353 | gi\|14042970 | NP_114428.1 | protein ITFG3 [Homo sapiens] |
| 1354 | gi\|21362050 | NP_115733.2 | coiled-coil domain-containing protein 115 [Homo sapiens] |
| 1355 | gi\|160420328 | NP_919255.2 | iron-sulfur cluster assembly 2 homolog, mitochondrial precursor [Homo sapiens] |
| 1356 | gi\|289577080 | NP_001409.3 | eukaryotic translation initiation factor 4 gamma 2 isoform 1 [Homo sapiens] |
| 1357 | gi\|61835204 | NP_001013454.1 | 3-mercaptopyruvate sulfurtransferase isoform 2 [Homo sapiens] |
| 1358 | gi\|6681764 | NP_004993.1 | NADH dehydrogenase [ubiquinone] 1 alpha subcomplex subunit 9, mitochondrial precursor [Homo sapiens] |
| 1359 | gi\|106049292 | NP_071504.2 | pyruvate carboxylase, mitochondrial precursor [Homo sapiens] |
| 1360 | gi\|156416003 | NP_004159.2 | succinate dehydrogenase [ubiquinone] flavoprotein subunit, mitochondrial precursor [Homo sapiens] |
| 1361 | gi\|8922116 | NP_060056.1 | calcium channel flower homolog isoform a [Homo sapiens] |
| 1362 | gi\|166197690 | NP_001107562.1 | ephexin-1 isoform 2 [Homo sapiens] |
| 1363 | gi\|75677343 | NP_056359.2 | transmembrane protein 98 [Homo sapiens] |
| 1364 | gi\|187829452 | NP_001120703.1 | autism susceptibility gene 2 protein isoform 2 [Homo sapiens] |
| 1365 | gi\|21361763 | NP_061115.2 | transcription cofactor HES-6 isoform a [Homo sapiens] |
| 1366 | gi\|91807119 | NP_079417.2 | TM2 domain-containing protein 3 isoform b [Homo sapiens] |
| 1367 | gi\|38327632 | NP_001945.3 | epithelial discoidin domain-containing receptor 1 isoform 1 precursor [Homo sapiens] |
| 1368 | gi\|14165437 | NP_112553.1 | heterogeneous nuclear ribonucleoprotein K isoform a [Homo sapiens] |
| 1369 | gi\|313760624 | NP_000433.4 | platelet endothelial cell adhesion molecule precursor [Homo sapiens] |
| 1370 | gi\|25777615 | NP_002802.2 | 26S proteasome non-ATPase regulatory subunit 7 [Homo sapiens] |
| 1371 | gi\|5453990 | NP_006254.1 | proteasome activator complex subunit 1 isoform 1 [Homo sapiens] |
| 1372 | gi\|4506641 | NP_000988.1 | 60S ribosomal protein L37 [Homo sapiens] |
| 1373 | gi\|4506711 | NP_001021.1 | 40S ribosomal protein S27 [Homo sapiens] |
| 1374 | gi\|21396500 | NP_003600.2 | HIRA-interacting protein 3 [Homo sapiens] |
| 1375 | gi\|25777596 | NP_003639.2 | diacylglycerol kinase delta isoform 1 [Homo sapiens] |
| 1376 | gi\|52486265 | NP_006020.4 | paraneoplastic antigen Ma1 [Homo sapiens] |
| 1377 | gi\|57242774 | NP_055495.2 | hypothetical protein LOC9703 precursor [Homo sapiens] |
| 1378 | gi\|151108509 | NP_055674.4 | rho GTPase-activating protein 44 [Homo sapiens] |
| 1379 | gi\|104876423 | NP_006031.2 | heparan sulfate glucosamine 3-0-sulfotransferase 4 [Homo sapiens] |
| 1380 | gi\|11641421 | NP_067090.1 | LAG1 longevity assurance homolog 1 isoform 1 [Homo sapiens] |
| 1381 | gi\|166795250 | NP_006836.2 | kinesin-like protein KIF2C [Homo sapiens] |
| 1382 | gi\|6912440 | NP_036411.1 | zinc finger protein 346 [Homo sapiens] |
| 1383 | gi\|17999541 | NP_060676.2 | vacuolar protein sorting-associated protein 35 [Homo sapiens] |
| 1384 | gi\|17978481 | NP_536338.1 | vacuolar protein sorting-associated protein 16 homolog isoform 3 [Homo sapiens] |
| 1385 | gi\|18079254 | NP_110442.1 | p53 and DNA damage-regulated protein 1 [Homo sapiens] |
| 1386 | gi\|22035616 | NP_665741.1 | oxysterol-binding protein-related protein 7 [Homo sapiens] |
| 1387 | gi\|86198310 | NP_001034444.1 | mitochondrial carnitine/acylcarnitine carrier protein CACL [Homo sapiens] |
| 1388 | gi\|194097323 | NP_004083.3 | enoyl-CoA hydratase, mitochondrial precursor [Homo sapiens] |
| 1389 | gi\|193785653 | BAG51088.1 | unnamed protein product [Homo sapiens] |
| 1390 | gi\|4507801 | NP_003343.1 | small ubiquitin-related modifier 1 isoform a precursor [Homo sapiens] |
| 1391 | gi\|4503513 | NP_003748.1 | eukaryotic translation initiation factor 3 subunit I [Homo sapiens] |
| 1392 | gi\|19923191 | NP_003897.2 | 80 kDa MCM3-associated protein [Homo sapiens] |
| 1393 | gi\|195972859 | NP_004219.3 | cytohesin-2 isoform 2 [Homo sapiens] |
| 1394 | gi\|8392875 | NP_037374.1 | transcription factor IIB [Homo sapiens] |
| 1395 | gi\|56676379 | NP_057088.2 | intraflagellar transport protein 52 homolog [Homo sapiens] |
| 1396 | gi\|261490712 | NP_612408.3 | transmembrane protein 44 isoform a [Homo sapiens] |
| 1397 | gi\|4502303 | NP_001688.1 | ATP synthase subunit O, mitochondrial precursor [Homo sapiens] |
| 1398 | gi\|115583663 | NP_000925.2 | alpha-2-antiplasmin isoform a precursor [Homo sapiens] |
| 1399 | gi\|4506061 | NP_002724.1 | 5'-AMP-activated protein kinase subunit gamma-1 isoform 1 [Homo sapiens] |
| 1400 | gi\|47132589 | NP_002732.3 | serine/threonine-protein kinase N1 isoform 2 [Homo sapiens] |
| 1401 | gi\|4557871 | NP_001054.1 | serotransferrin precursor [Homo sapiens] |
| 1402 | gi\|4507521 | NP_001055.1 | transketolase [Homo sapiens] |
| 1403 | gi\|4758220 | NP_004690.1 | XAP-5 protein [Homo sapiens] |
| 1404 | gi\|115527097 | NP_006026.3 | serine/threonine-protein kinase MRCK beta [Homo sapiens] |
| 1405 | gi\|5031595 | NP_005709.1 | actin-related protein 2/3 complex subunit 4 isoform a [Homo sapiens] |
| 1406 | gi\|166235178 | NP_001107579.1 | PDZ and LIM domain protein 3 isoform b [Homo sapiens] |
| 1407 | gi\|7705851 | NP_057223.1 | coiled-coil-helix-coiled-coil-helix domain-containing protein 2, mitochondrial precursor [Homo sapiens] |
| 1408 | gi\|170932471 | NP_078926.3 | putative uncharacterized protein C11orf80 [Homo sapiens] |
| 1409 | gi\|13654276 | NP_112486.1 | queuine tRNA-ribosyltransferase [Homo sapiens] |
| 1410 | gi\|54607110 | NP_777589.2 | zinc finger MIZ domain-containing protein 2 isoform 2 [Homo sapiens] |
| 1411 | gi\|58761496 | NP_001011724.1 | heterogeneous nuclear ribonucleoprotein A1-like 2 [Homo sapiens] |
| 1412 | gi\|117956373 | NP_001071153.1 | arf-GAP with GTPase, ANK repeat and PH domain-containing protein 7 [Homo sapiens] |
| 1413 | gi\|50301238 | NP_000628.2 | glutathione reductase, mitochondrial isoform 1 precursor [Homo sapiens] |
| 1414 | gi\|48255891 | NP_001001329.1 | glucosidase 2 subunit beta isoform 2 [Homo sapiens] |
| 1415 | gi\|4506887 | NP_003000.1 | selenoprotein W [Homo sapiens] |
| 1416 | gi\|6102858 | CAB59243.1 | hypothetical protein [Homo sapiens] |
| 1417 | gi\|16554616 | NP_115865.1 | 28S ribosomal protein S6, mitochondrial [Homo sapiens] |
| 1418 | gi\|4502313 | NP_001685.1 | V-type proton ATPase 16 kDa proteolipid subunit [Homo sapiens] |
| 1419 | gi\|13259510 | NP_004073.2 | dynactin subunit 1 isoform 1 [Homo sapiens] |
| 1420 | gi\|55750041 | NP_001931.2 | atrophin-1 [Homo sapiens] |
| 1421 | gi\|4504111 | NP_002077.1 | growth factor receptor-bound protein 2 isoform 1 [Homo sapiens] |
| 1422 | gi\|194018520 | NP_002085.2 | eukaryotic peptide chain release factor GTP-binding subunit ERF3A isoform 1 [Homo sapiens] |
| 1423 | gi\|31542947 | NP_002147.2 | 60 kDa heat shock protein, mitochondrial [Homo sapiens] |
| 1424 | gi\|116829964 | NP_002247.3 | metastasis-suppressor KiSS-1 [Homo sapiens] |
| 1425 | gi\|153945728 | NP_005900.2 | microtubule-associated protein 1B [Homo sapiens] |
| 1426 | gi\|8400715 | NP_058525.1 | microtubule-associated protein tau isoform 4 [Homo sapiens] |
| 1427 | gi\|14043022 | NP_004981.2 | methionyl-tRNA synthetase, cytoplasmic [Homo sapiens] |
| 1428 | gi\|4758954 | NP_004567.1 | serine/threonine-protein phosphatase 2A 55 kDa regulatory subunit B beta isoform isoform a [Homo sapiens] |
| 1429 | gi\|22538467 | NP_002787.2 | proteasome subunit beta type-4 [Homo sapiens] |
| 1430 | gi\|48762926 | NP_005040.2 | periodic tryptophan protein 2 homolog [Homo sapiens] |
| 1431 | gi\|14141193 | NP_001004.2 | 40S ribosomal protein S9 [Homo sapiens] |
| 1432 | gi\|171846268 | NP_005717.3 | elongation factor Ts, mitochondrial isoform 2 precursor [Homo sapiens] |
| 1433 | gi\|5031599 | NP_005722.1 | actin-related protein 2/3 complex subunit 2 [Homo sapiens] |
| 1434 | gi\|5032179 | NP_005753.1 | transcription intermediary factor 1-beta [Homo sapiens] |
| 1435 | gi\|37655183 | NP_006087.2 | protein NDRG1 [Homo sapiens] |
| 1436 | gi\|22907052 | NP_006400.2 | actin-related protein 2/3 complex subunit 1A isoform 1 [Homo sapiens] |
| 1437 | gi\|11055017 | AAG27922.1 | hypothetical protein SBBl23 [Homo sapiens] |
| 1438 | gi\|112421108 | NP_055940.3 | protein capicua homolog [Homo sapiens] |
| 1439 | gi\|6679189 | NP_031390.1 | transmembrane emp24 domain-containing protein 3 precursor [Homo sapiens] |
| 1440 | gi\|46367787 | NP_002559.2 | polyadenylate-binding protein 1 [Homo sapiens] |
| 1441 | gi\|9910352 | NP_064530.1 | endophilin-B2 [Homo sapiens] |
| 1442 | gi\|16445029 | NP_443100.1 | immunoglobulin superfamily member 8 [Homo sapiens] |
| 1443 | gi\|16945972 | NP_476502.1 | kelch domain-containing protein 3 isoform 1 [Homo sapiens] |
| 1444 | gi\|28274701 | NP_665805.2 | zinc finger protein 511 [Homo sapiens] |
| 1445 | gi\|4505489 | NP_002530.1 | ornithine decarboxylase [Homo sapiens] |
| 1446 | gi\|4506671 | NP_000995.1 | 60S acidic ribosomal protein P2 [Homo sapiens] |
| 1447 | gi\|4506675 | NP_002941.1 | dolichyl-diphosphooligosaccharide--protein glycosyltransferase subunit 1 precursor [Homo sapiens] |
| 1448 | gi\|194595509 | NP_001123910.1 | spectrin alpha chain, brain isoform 1 [Homo sapiens] |
| 1449 | gi\|13236579 | NP_077306.1 | long-chain fatty acid transport protein 3 [Homo sapiens] |
| 1450 | gi\|215277017 | NP_001135842.1 | tyrosine-protein phosphatase non-receptor type 18 isoform 2 [Homo sapiens] |
| 1451 | gi\|194018570 | NP_060388.2 | coiled-coil domain-containing protein 109B [Homo sapiens] |
| 1452 | gi\|8922794 | NP_060755.1 | U3 small nucleolar ribonucleoprotein protein IMP3 [Homo sapiens] |
| 1453 | gi\|209976986 | NP_060856.2 | PCl domain-containing protein 2 [Homo sapiens] |
| 1454 | gi\|113722120 | NP_115495.3 | G-protein coupled receptor 98 precursor [Homo sapiens] |
| 1455 | gi\|10438605 | BAB15287.1 | unnamed protein product [Homo sapiens] |
| 1456 | gi\|27735039 | NP_775744.1 | anaphase-promoting complex subunit 16 isoform 1 [Homo sapiens] |
| 1457 | gi\|5031857 | NP_005557.1 | L-lactate dehydrogenase A chain isoform 1 [Homo sapiens] |
| 1458 | gi\|14505021 | NP_002328.1 | alpha-2-macroglobulin receptor-associated protein precursor [Homo sapiens] |
| 1459 | gi\|6631095 | NP_002379.2 | DNA replication licensing factor MCM3 [Homo sapiens] |
| 1460 | gi\|28193182 | CAD62333.1 | unnamed protein product [Homo sapiens] |
| 1461 | gi\|33598948 | NP_002651.2 | 1-phosphatidylinositol-4,5-bisphosphate phosphodiesterase gamma-1 isoform a [Homo sapiens] |
| 1462 | gi\|223468676 | NP_068810.3 | transcription factor p65 isoform 1 [Homo sapiens] |
| 1463 | gi\|49640009 | NP_003307.3 | E3 ubiquitin-protein ligase TTC3 [Homo sapiens] |
| 1464 | gi\|22027528 | NP_663788.1 | rho guanine nucleotide exchange factor 7 isoform b [Homo sapiens] |
| 1465 | gi\|83716024 | NP_060066.2 | kinesin-like protein KIF21B [Homo sapiens] |
| 1466 | gi\|109148508 | NP_060140.2 | ubiquitin thioesterase OTUB1 [Homo sapiens] |
| 1467 | gi\|11034855 | NP_065695.1 | transmembrane protein 9B precursor [Homo sapiens] |
| 1468 | gi\|50345991 | NP_001001975.1 | ATP synthase subunit delta, mitochondrial precursor [Homo sapiens] |
| 1469 | gi\|4557499 | NP_001320.1 | C-terminal-binding protein 2 isoform 1 [Homo sapiens] |
| 1470 | gi\|4758346 | NP_004098.1 | lgG receptor FcRn large subunit p51 precursor [Homo sapiens] |
| 1471 | gi\|51476154 | CAH18067.1 | hypothetical protein [Homo sapiens] |
| 1472 | gi\|7657196 | NP_055302.1 | zinc finger protein 330 [Homo sapiens] |
| 1473 | gi\|156416005 | NP_054905.2 | COMM domain-containing protein 9 isoform 1 [Homo sapiens] |

## Patentansprüche

1. Anordnung von Brustkrebs-spezifischen Markersequenzen auf einem Träger zur Früherkennung, Diagnose, Prognose, Therapiesteuerung bei Brustkrebs mit einem oder mehreren verschiedenen Brustkrebs-spezifischen Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID. No. 1 - 982.

2. Anordnung von Brustkrebs-spezifischen Markersequenzen auf einem Träger zur Analyse von Brustkrebs-spezifischen Autoantikörperprofilen mit einem oder mehreren Brustkrebs-spezifischen Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID. No. 1 - 982.

3. Anordnung nach einem der vorgehenden Ansprüche wobei 2 oder 3, vorzugsweise 4 oder 5, besonders bevorzugt 7 oder 8 oder mehr unterschiedliche Brustkrebs-spezifische Markersequenzen umfasst sind.

4. Anordnung nach einem der vorgehenden Ansprüche wobei mindestens 2 bis 5 oder 10, vorzugsweise 30 bis 50 Brustkrebs-spezifische Markersequenzen oder 50 bis 100 oder mehr Brustkrebs-spezifische Markersequenzen an oder zu einem zu untersuchenden Patienten bestimmt werden.

5. Anordnung nach einem der vorgehenden Ansprüche, **dadurch gekennzeichnet, dass** die Brustkrebs-spezifischen Markersequenzen auf einem festen Träger aufgebracht werden, insbesondere einem Filter, einer Membran, einem magnetischen oder Fluorophor-markierten Kügelchen, einem Silizium-Wafer, Glas, Metall, Kunststoff, einem Chip, einem massenspektrometrisches Target oder einer Matrix.

6. Anordnung nach einem der vorgehenden Ansprüche, **dadurch gekennzeichnet, dass** die Brustkrebs-spezifischen Markersequenzen als Klone vorliegen.

7. Assay oder Proteinbiochip umfassend eine Anordnung nach einem der Ansprüche 1 bis 6.

8. Verwendung einer Anordnung nach einem der Ansprüche 1 bis 6 oder eines Assays oder Proteinbiochips nach Anspruch 7 zur Analyse von Autoantikörperprofilen von Patienten, insbesondere zur quantitativen Analyse und/oder zur Überwachung von Veränderungen von Autoantikörperprofilen von Patienten.

9. Diagnostikum (Test Kit) zur Früherkennung und/oder Diagnose von Brustkrebs und/oder Prognose und/oder Vorhersage des Risikos der Metastasenbildung bei Brustkrebs umfassend eine Anordnung nach einem der Ansprüche 1 bis 6 oder einen Assay oder Proteinbiochip nach Anspruch 7 und gegebenenfalls weitere Zusatz- und Hilfsstoffe.

10. Diagnostikum (Test Kit) zur Therapieüberwachung und/oder Nachsorge bei Brustkrebs umfassend eine Anordnung nach einem der Ansprüche 1 bis 6 oder einen Assay oder Proteinbiochip nach Anspruch 7 und gegebenenfalls weitere Zusatz- und Hilfsstoffe.

11. Verfahren zur Früherkennung und Diagnose von Brustkrebs wobei
a.) eine oder mehrere Brustkrebs-spezifische Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID No. 1 - 982 auf einem Träger aufgebracht werden und
b.) mit Körperflüssigkeit oder Gewebeauszug eines Patienten in Kontakt gebracht werden und
c.) der Nachweis einer Wechselwirkung der Körperflüssigkeit oder Gewebeauszug mit den Brustkrebs-spezifischen Markersequenzen aus a.) erfolgt.

12. Verfahren zum Stratifizieren, insbesondere zur Risikostratifizierung, oder zur Therapiesteuerung eines Patienten mit Brustkrebs, wobei mit Hilfe einer oder mehrerer Brustkrebs-spezifischer Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID No. 1 - 982 das Autoantikörperprofil eines Patienten bestimmt und gegebenenfalls überwacht wird.

13. Verfahren nach Anspruch 12, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

14. Target zur Behandlung und Therapie von Brustkrebs ausgewählt aus der Gruppe der Proteine SEQ ID No. 983 bis 1473, Teilsequenzen dieser Proteine und den für diese Proteine kodierenden Sequenzen, den Sequenzen SEQ ID No. 1 - 491 und SEQ ID. No. 492 - 982 und Teilsequenzen von SEQ ID No 1 - 982 sowie der von SEQ ID No. 1 - 982 kodierten Proteine.

15. Verwendung einer Brustkrebs-spezifischen Markersequenz ausgewählt aus der Gruppe der Proteine SEQ ID No. 983 bis 1473, Teilsequenzen dieser Proteine und den für diese Proteine kodierenden Sequenzen, den Sequenzen SEQ ID No. 1 - 491 und SEQ ID. No. 492 - 982 und Teilsequenzen von SEQ ID No 1 - 982 sowie der von SEQ ID No. 1 - 982 kodierten Proteine als Affinitätsmaterial zur Durchführung einer Apherese oder Blutwäsche für Patienten mit Brustkrebs.

16. Verfahren zur Identifizierung von Brustkrebs-spezifischen Markersequenzen umfassend die Schritte
a. Bereitstellung von Markersequenzen auf einem Array,
b. Identifizierung von Brustkrebs-spezifischen Markersequenzkandidaten durch vergleichende Analyse der Signale, die bei Kontakt der Markersequenzen mit
Körperflüssigkeit oder Gewebeauszug eines Patienten mit Brustkrebs und
Körperflüssigkeit oder Gewebeauszug eines Patienten ohne Brustkrebs gemessen werden,
c. Charakterisierung der Brustkrebs-spezifischen Markersequenzkandidaten mit Hilfe eines Proteinbiochips,
d. Auswahl von Brustkrebs-spezifischen Markersequenzkandidaten, die ein unterschiedliches Signal bei Patienten mit Brustkrebs und Patienten ohne Brustkrebs liefern (Brustkrebs-spezifische Markersequenzen).

17. Verwendung von einer oder mehreren Brustkrebs-spezifischen Markersequenzen ausgewählt aus der Gruppe der Proteine SEQ ID No. 983 bis 1473 und/oder Teilsequenzen dieser Proteine und/oder kodiert durch Sequenzen SEQ ID No. 1 - 491 und/oder kodiert durch Sequenzen SEQ ID. No. 492 - 982 und/oder kodiert durch Teilsequenzen von SEQ ID. No. 1 - 982 für Brustkrebsdiagnose und Therapie, insbesondere zur Früherkennung von Brustkrebs, Diagnose von Brustkrebs, Prognose, beispielsweise des Risikos der Metastasenbildung, Therapiesteuerung, beispielsweise Vorhersage und Überwachung des Ansprechens auf ein Arzneimittel oder eine Therapie, Nachsorge.

18. Verwendung einer Anordnung nach einem der Ansprüche 1 bis 6 oder eines Assays oder Proteinbiochips nach Anspruch 7 zum Screenen von Substanzen (Wirkstoffen) für Brustkrebs.

19. Verfahren zur Identifizierung und/oder Charakterisierung einer Substanz (Wirkstoff)für Brustkrebs wobei
a) eine oder mehrere Brustkrebs-spezifische Markersequenzen ausgewählt aus der Gruppe der Proteine SEQ ID No. 983 bis 1473 und/oder Teilsequenzen dieser Proteine und/oder der Sequenzen SEQ ID No. 1 - 491 und/oder der Sequenzen SEQ ID. No. 492 - 982 und/oder Teilsequenzen von SEQ ID No. 1 - 982 und/oder der durch die Nukeinsäuresequenzen kodierten Proteine
b) mit mindestens einer zu untersuchenden Substanz in Kontakt gebracht werden,
c) ein Bindungserfolg nachgewiesen wird.
